Europäisches Patentamt
European Patent Office
Office européen des brevets

Veröffentlichungsnummer: **0 000 485**
A1

# EUROPÄISCHE PATENTANMELDUNG

Anmeldenummer: 78100308.2

Anmeldetag: 05.07.78

Int. Cl.²: C 07 D 401/14, A 61 K 31/00 //C07D213/69, C07D209/08, C07D213/65

Priorität: 08.07.77 CH 8483/77
14.06.78 CH 6488/78

Veröffentlichungstag der Anmeldung:
07.02.79 Bulletin 79/3

Benannte Vertragsstaaten:
BE CH DE FR GB NL SE

Anmelder: CIBA-GEIGY AG
Patentabteilung Postfach
CH-4002 Basel. (CH)

Erfinder: Eichenberger, Kurt, Dr. Dr.
Neubergweg 36
CH-4106 Therwil. (CH)

Erfinder: Kühnis, Hans, Dr.
Lange Gasse 26
CH-4052 Basel. (CH)

Erfinder: Ostermayer, Franz, Dr.
Am Hang 5
CH-4125 Riehen. (CH)

Erfinder: Schröter, Herbert, Dr.
Arisdörferstrasse 16
CH-4414 Füllinsdorf. (CH)

**Piperidino-propanole, ihre Herstellung und pharmazeutische Präparate die diese enthalten.**

Die Erfindung betrifft neue Piperidino-propanole, insbesondere 1-(3-Heterocyclyloxy -2-hydroxy- propanol)-4-(N-diazacyclyl)- piperidine der Formel

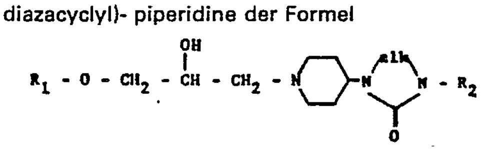

(I)

worin $R_1$ einen gegebenenfalls substituierten Heteroarylrest darstellt, $R_2$ Wasserstoff oder einen gegebenenfalls substituierten aliphatischen, cycloaliphatischen, cycloaliphatisch-aliphatischen oder araliphatischen Kohlenwasserstoffrest oder einen Acylrest bedeutet, und alk für Niederalkylen steht, das die beiden Stickstoffatome durch 2 oder 3 Kohlenstoffatome voneinander trennt, oder für einen gegebenenfalls substituierten 1,2-Phenylenrest steht, und deren Salze, sowie Verfahren zu ihrer Herstellung, ferner pharmazeutische Präparate enthaltend diese Verbindungen and ihre Verwendung, vorzugsweise in Form von pharmazeutischen Präparaten als Antihypertensiva, Antitachycardica und α-Sympathicolytica.

EP 0 000 485 A1

Croydon Printing Company Ltd.

CIBA-GEIGY AG
Patentabteilung

Case 4-11240/+

BEZEICHNUNG GEÄNDERT
siehe Titelseite

Piperidino-propanole

Die Erfindung betrifft neue Piperidino-propanole, insbesondere 1-(3-Heterocyclyloxy-2-hydroxy-propanol)-4-(N-diazacyclyl)-piperidine der Formel

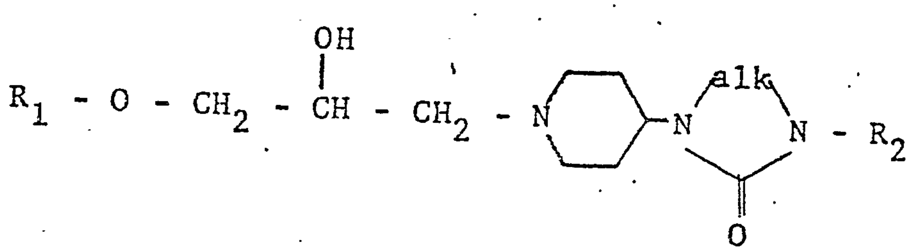

(I)

worin $R_1$ einen gegebenenfalls substituierten Heteroarylrest darstellt, $R_2$ Wasserstoff oder einen gegebenenfalls substituierten aliphatischen, cycloaliphatischen, cycloaliphatisch-aliphatischen oder araliphatischen Kohlenwasserstoffrest oder einen Acylrest bedeutet, und alk für Niederalkylen steht, das die beiden Stickstoffatome durch 2 oder 3 Kohlenstoffatome voneinander trennt, oder für einen gegebenenfalls substituierten 1,2-Phenylenrest steht, und deren Salze, sowie Verfahren zu ihrer Herstellung, ferner pharmazeutische Präparate enthaltend diese Verbindungen und ihre Verwendung, vorzugsweise in Form von pharmazeutischen Präparaten.

Ein Heteroarylrest $R_1$ ist ein gegebenenfalls substituierter, vorzugsweise monocyclischer sowie bicyclischer Heteroarylrest, in erster Linie ein gegebenenfalls, z.B. einfach, zweifach oder mehrfach, sub-

BAD ORIGINAL

stituierter, vorzugsweise monocyclischer Azaarylrest mit 5 bis 6 Ringgliedern und 1 bis 2 Ringstickstoffatomen, wie gegebenenfalls substituiertes Pyridyl, z.B. 2-, 3- oder 4-Pyridyl, Imidazolyl, z.B. 2-Imidazolyl, Pyrimidinyl, z.B. 2- oder 4-Pyrimidinyl, Pyridazinyl, z.B. 2-Pyridazinyl, oder Pyrazinyl, z.B. 2-Pyrazinyl, ferner ein gegebenenfalls substituierter bicyclischer Azaarylrest, insbesondere Benzoazaarylrest, mit 5 bis 6 Ringgliedern und 1 bis 2 Ringstickstoffatomen im Azaarylrest, wie gegegebenenfalls substituiertes Indolyl, z.B. 4-Indolyl, Chinolinyl, z.B. 4-Chinolinyl, oder Isochinolinyl, z.B. 1-Isochinolinyl. Substituenten eines Heteroarylrestes sind u.a. gegebenenfalls substituierte aliphatische oder aromatische Kohlenwasserstoffreste, gegebenenfalls veräthertes oder verestertes Hydroxy oder Mercapto, Acyl, Nitro oder gegebenenfalls substituiertes Amino.

Gegebenenfalls substituierte aliphatische Kohlenwasserstoffreste $R_2$ sind entsprechendes Niederalkyl, sowie Niederalkenyl oder Niederalkinyl, wobei Substituenten, insbesondere von Niederalkyl, in erster Linie gegebenenfalls veräthertes oder verestertes Hydroxy oder Mercapto, Acyl oder gegebenenfalls substituiertes Amino sind.

Gegebenenfalls substituierte cycloaliphatische und cycloaliphatisch-aliphatische Kohlenwasserstoffreste $R_2$ sind Cycloalkyl, vorzugsweise mit 3-8, insbesondere 5-7 Ringkohlenstoffatomen, oder Cycloalkenyl, vorzugsweise mit 5-8, insbesondere 6 oder 7 Ringkohlenstoffatomen, ferner Cycloalkylniederalkyl, sowie Cycloalkenylniederalkyl, worin Cycloalkyl bzw. Cycloalkenyl die oben gegebenen Bedeutungen haben. Substituenten von cycloaliphatischen und cycloaliphatisch-aliphatischen Kohlenwasserstoffresten sind in erster Linie gegebenenfalls substituierte aliphatische Kohlenwasserstoffreste, gegebenenfalls veräthertes oder verestertes Hydroxy oder Oxo.

Araliphatische Kohlenwasserstoffreste $R_2$ sind in erster Linie gegebenenfalls substituiertes Phenylniederalkyl, worin Phenyl als Substituenten z.B. gegebenenfalls substituierte aliphatische Kohlenwasserstoffreste, gegebenenfalls veräthertes Hydroxy oder Mercapto, Acyl, Nitro oder gegebenenfalls substituiertes Amino enthalten kann.

Gegebenenfalls substituierte aliphatische oder aromatische Kohlenwasserstoffreste als Substituenten eines Heteroarylrestes $R_1$ bzw. von cycloaliphatischen, cycloaliphatisch-aliphatischen oder araliphatischen Kohlenwasserstoffresten $R_2$ enthalten u.a. gegebenenfalls verätherte oder veresterte Hydroxygruppen oder gegebenenfalls substituiertes Amino als Substituenten.

Veräthertes Hydroxy ist insbesondere Niederalkoxy oder Phenylniederalkoxy, ferner Niederalkenyloxy oder Niederalkinyloxy, sowie Hydroxyniederalkoxy, Niederalkoxyniederalkoxy, Niederalkylthioniederalkoxy oder Niederalkanoylniederalkoxy, während verestertes Hydroxy insbesondere Halogen, ferner Niederalkanoyloxy darstellt.

Veräthertes Mercapto ist in erster Linie Niederalkylthio, während veresterte Mercapto z.B. Niederalkanoylthio ist.

Acyl ist vorzugsweise der entsprechende Rest einer organischen Carbonsäure und steht insbesondere für Niederalkanoyl. Acyl steht ebenfalls für den entsprechenden Rest eines Kohlensäurehalbderivats, wie für Niederalkoxycarbonyl oder gegebenenfalls substituiertes Carbamoyl. Ein Acylrest im weitesten Sinn der Definition ist auch Cyan.

Gegebenenfalls substituiertes Amino ist Acyl-

BAD ORIGINAL

amino, insbesondere Niederalkanoylamino oder Niederalkoxycarbonylamino, ferner gegebenenfalls substituiertes Ureido. Substituiertes Amino ist ebenfalls Niederalkylamino oder Diniederalkylamino, sowie Niederalkylenamino, Niederoxaalkylenamino oder Niederazaalkylenamino, wobei in letzterem der Azastickstoff vorzugsweise, z.B. durch Niederalkyl, substituiert ist.

Niederalkylen alk ist vorzugsweise unverzweigtes Niederalkylen und in erster Linie Aethylen, sowie 1,3-Propylen, kann aber auch verzweigtes Niederalkylen, wie 1,2-Propylen, 1,2- oder 2,3-Butylen sein.

Wenn alk für den 1,2-Phenylenrest steht, so kann dieser durch Niederalkyl, Niederalkoxy oder Halogen substituiert sein.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Niederalkyl ist z.B. Methyl, Aethyl, n-Propyl, Isopropyl, n-Butyl, Isobutyl oder tert.-Butyl; substituiertes Niederalkyl ist insbesondere entsprechendes Methyl oder 1- oder 2-Aethyl.

Niederalkenyl ist z.B. Vinyl, Allyl, 2- oder 3-Methallyl oder 3,3-Dimethylallyl.

Niederalkinyl ist insbesondere Propargyl.

Cycloalkyl ist in erster Linie Cyclopentyl, Cyclohexyl oder Cycloheptyl, ferner Cyclopropyl oder Cyclooctyl.

Cycloalkenyl ist z.B. 1- oder 3-Cyclohexenyl oder 1-Cycloheptenyl.

Cycloalkylniederalkyl ist z.B. Cyclopentylmethyl, Cyclohexylmethyl, 2-Cyclohexyläthyl oder Cycloheptylmethyl.

Cycloalkenylniederalkyl steht z.B. für 1-Cyclohexenylmethyl, 2-(1-Cyclohexenyl)-äthyl oder 1-Cycloheptenylmethyl.

Phenylniederalkyl ist u.a. Benzyl, 1- oder 2-Phenyläthyl oder 3-Phenylpropyl.

Niederalkoxy ist z.B. Methoxy, Aethoxy, n-Propyloxy, Isopropyloxy, n-Butyloxy oder Isobutyloxy.

Phenylniederalkoxy ist z.B. Benzyloxy oder 1- oder 2-Phenyläthoxy.

Hydroxyniederalkoxy ist z.B. 2-Hydroxyäthoxy, ferner 2- oder 3-Hydroxypropyloxy.

Niederalkoxyniederalkoxy ist u.a. Niederalkoxymethoxy oder 1- und insbesondere 2-Niederalkoxy-äthoxy, z.B. Methoxy-methoxy, 2-Methoxy-äthoxy oder 2-Aethoxy-äthoxy.

Niederalkylthioniederalkoxy ist insbesondere Niederalkylthiomethoxy oder 1- und in erster Linie 2-Niederalkylthio-äthoxy, z.B. 2-Methylthio-äthoxy oder 2-Aethylthio-äthoxy.

Niederalkanoylniederalkoxy ist insbesondere Acetonyloxy.

Niederalkenyloxy ist z.B. Allyloxy, 2- oder 3-Methallyloxy oder 3,3-Dimethylallyloxy.

Niederalkinyloxy ist insbesondere Propargyloxy.

Halogen ist vorzugsweise Halogen mit Atomnummer bis zu 35, d.h. Fluor, Chlor oder Brom.

Niederalkanoyloxy steht z.B. für Acetyloxy, Propionyloxy oder Pivaloyloxy.

Niederalkylthio ist z.B. Methylthio, Aethylthio, n-Propylthio oder Isopropylthio.

Niederalkanoylthio ist u.a. Acetylthio oder Propionylthio.

Niederalkanoyl ist z.B. Acetyl, Propionyl oder Butyryl.

Niederalkoxycarbonyl ist z.B. Methoxycarbonyl oder Aethoxycarbonyl.

Gegebenenfalls substituiertes Carbamoyl ist z.B. Carbamoyl, oder N-Niederalkyl- oder N,N-Diniederalkylcarbamoyl, wie N-Methyl-carbamoyl, N,N-Dimethylcarbamoyl, N-Aethylcarbamoyl oder N,N-Diäthylcarbamoyl.

Niederalkanoylamino ist z.B. Acetylamino oder Propionylamino.

Niederalkoxycarbonylamino ist z.B. Methoxycarbonylamino oder Aethoxycarbonylamino.

Gegebenenfalls substituiertes Ureido ist z.B. Ureido oder 3-Niederalkyl- oder 3-Cycloalkyl-ureido, worin Cycloalkyl z.B. 5-7 Ringglieder hat, z.B. 3-Methylureido, 3-Aethylureido oder 3-Cyclohexylureido.

N-Niederalkylamino und N,N-Diniederalkylamino sind z.B. Methylamino, Aethylamino, Dimethylamino oder Diäthylamino.

Niederalkylenamino enthält vorzugsweise 5-7 Ringkohlenstoffatome und ist z.B. Pyrrolidino oder Piperidino.

Niederoxaalkylenamino ist in erster Linie Morpholino, während Niederazaalkylenamino insbesondere entsprechendes N-Niederalkyl-niederazaalkylenamino, z.B. 4-Methyl-1-piperazino, ist.

Unter den substituierten Niederalkylgruppen sind z.B. Hydroxyniederalkyl, Niederalkoxyniederalkyl, Halogenniederalkyl, Niederalkanoylaminoniederalkyl oder Niederalkoxycarbonylaminoniederalkyl zu nennen.

Hydroxyniederalkyl ist vorzugsweise Hydroxy-[illegible] 1- und [illegible] 2-Hydroxyäthyl.

Niederalkoxyniederalkyl ist vo[illegible]

BAD ORIGINAL

Niederalkoxymethyl oder 1- und in erster Linie 2-Niederalkoxyäthyl, z.B. Methoxymethyl, Aethoxymethyl, 2-Methoxy-äthyl oder 2-Aethoxy-äthyl.

Halogenniederalkyl ist vorzugsweise Halogenmethyl, z.B. Trifluormethyl.

Niederalkanoylaminoniederalkyl ist insbesondere Niederalkanoylaminomethyl oder 1- und in erster Linie 2-Niederalkanoylamino-äthyl, z.B. Acetylaminomethyl, 2-Acetylamino-äthyl oder 2-Propionylamino-äthyl.

Niederalkoxycarbonylaminoniederalkyl ist insbesondere Niederalkoxycarbonylaminomethyl, oder 1- und in erster Linie 2-Niederalkoxycarbonylamino-äthyl, z.B. Methoxycarbonylaminomethyl, 2-Methoxycarbonylamino-äthyl oder 2-Aethoxycarbonylamino-äthyl.

Die neuen Verbindungen können in Form ihrer Salze, wie ihrer Säureadditionssalze und in erster Linie ihrer pharmazeutisch verwendbaren, nicht-toxischen Säureadditionssalze vorliegen. Geeignete Salze sind z.B. solche mit anorganischen Säuren, wie Halogenwasserstoffsäuren, z.B. Chlorwasserstoffsäure oder Bromwasserstoffsäure, Schwefelsäuren, z.B. Schwefelsäure, oder Phosphorsäuren, oder mit organischen Säuren, wie aliphatischen, cycloaliphatischen, aromatischen oder heterocyclischen Carbon- oder Sulfonsäuren, z.B. Ameisen-, Essig-, Propion-, Bernstein-, Glykol-, Milch-, Aepfel-, Wein-, Zitronen-, Ascorbin-, Malein-, Hydroxymalein-, Brenztrauben-, Fumar-, Benzoe-, 4-Aminobenzoe-, Anthranil-, 4-Hydroxybenzoe-, Salicyl-, 4-Aminosalicyl-, Embon-, Methansulfon-, Aethansulfon-, 2-Hydroxyäthansulfon-, Aethylensulfon-, Toluolsulfon-, Naphthalinsulfon- oder Sulfanilsäure.

Die neuen Verbindungen der vorliegenden Erfindung können in Form von Gemischen von Isomeren, wie Racematen, oder von reinen Isomeren, z.B. optisch aktiven

Antipoden, vorliegen.

Die neuen Verbindungen besitzen wertvolle pharmakologische Eigenschaften. So zeigen sie eine blutdrucksenkende Wirkung, wie sich im Tierversuch, z.B. bei i.v. Gabe in Dosen von etwa 0,1 bis etwa 30 mg/kg an der narkotisierten Katze zeigen lässt. Zusätzlich bewirken die neuen Verbindungen eine Antitachycardie, wie sich ebenfalls im Tierversuch zeigen lässt, z.B. in <u>in vitro</u> Versuchen bei Konzentrationen von etwa 1 bis etwa 100 γ/ml am Meerschweinchen-Herzen (Langendorff-Präparat), und eine α-Sympathicolyse, z.B. in <u>in vitro</u> Versuchen bei Konzentrationen von etwa 0,01 bis etwa 10 γ/ml an der Ratte (isoliertes perfundiertes Mesenterialarterienpräparat; nach einer Modifiation der Methode von McGregor, J.Physiol., Bd. 177, S. 21 (1965)). Die neuen Verbindungen können daher als Antihypertensiva, Antitachycardica und α-Sympathicolytica verwendet werden. Ferner können die neuen Verbindungen als Ausgangs- oder Zwischenprodukte für die Herstellung anderer, insbesondere therapeutisch wirksamer, Verbindungen dienen.

Die Erfindung betrifft in erster Linie Verbindungen der Formel I, worin $R_1$ gegebenenfalls substituiertes, monocyclisches Heteroaryl oder Benzoheteroaryl mit 5 bis 6 Ringgliedern und 1 oder 2 Ringstickstoffatomen im Rest $R_1$ bedeutet, wobei Substituenten des heterocyclischen Arylrests gegebenenfalls substituiertes Niederalkyl, z.B. Niederalkyl, Niederalkoxyniederalkyl, Niederalkanoylaminoniederalkyl oder Niederalkoxycarbonylaminoniederalkyl oder gegebenenfalls entsprechend substituiertes [illegible] oder gegebenenfalls veräthertes oder [illegible] oder Mercapto, z.B. Niederalkoxy, Nieder-[illegible], [illegible] niederalkoxy, [illegible] Halogen, und/oder Nitro, [illegible]

BAD ORIGINAL

Wasserstoff oder gegebenenfalls substituiertes Niederalkyl, Cycloalkyl, Cycloalkylniederalkyl oder Phenylniederalkyl steht, wobei Substituenten dieser Reste z.B. gegebenenfalls substituiertes Niederalkyl, z.B. Niederalkyl, oder gegebenenfalls veräthertes oder verestertes Hydroxy oder Mercapto, z.B. Niederalkoxy, Niederalkylthio, und/oder Halogen, sind, oder ferner für Niederalkanoyl oder Niederalkoxycarbonyl steht, und alk die oben gegebene Bedeutung hat, und Salze, insbesondere Säureadditionssalze, in erster Linie pharmazeutisch verwendbare, nichttoxische Säureadditionssalze davon.

Die Erfindung betrifft insbesondere Verbindungen der Formel I, worin $R_1$ gegebenenfalls durch Niederalkyl, z.B. Methyl, Niederalkoxy, z.B. Methoxy, Niederalkylthio, z.B. Methylthio oder Aethylthio, Halogen mit Atomnummer bis zu 35, z.B. Chlor oder Brom, und/oder Nitro, substituiertes monocyclisches Monoazaaryl oder Diazaaryl mit sechs Ringgleidern, wie Pyridyl, z.B. 2-, 3- oder 4-Pyridyl, Pyrimidinyl, z.B. 2- oder 4-Pyrimidinyl, Pyrazinyl, z.B. 2-Pyrazinyl oder Indolyl, z.B. 4-Indolyl darstellt, $R_2$ für Wasserstoff, Niederalkyl, z.B. Methyl, Aethyl oder Isopropyl, Cycloalkyl, z.B. Cyclopentyl oder gegebenenfalls im Phenylteil durch Niederalkyl, z.B. Methyl, Niederalkoxy, z.B. Methoxy, und/oder Halogen mit Atomnummer bis zu 35, z.B. Chlor oder Brom, substituiertes Phenylniederalkyl, z.B. Benzyl oder 1- oder 2-Phenyläthyl, steht, und alk Niederalkylen mit 2-3 Kohlenstoffatomen bedeutet, das die beiden Stickstoffatome durch 2-3 Kohlenstoffatome trennt, z.B. Aethylen oder 1,3-Propylen, und Salze, insbesondere Säureadditionssalze, in erster Linie pharmazeutisch verwendbare nicht-toxische Säureadditionssalze davon.

Die Erfindung betrifft insbesondere Verbindungen der Formel I, worin $R_1$ für gegebenenfalls durch Nieder-

alkyl, z.B. durch Methyl, Niederalkoxy, z.B. Methoxy, Niederalkylthio, z.B. Methylthio oder Aethylthio, Halogen mit Atomnummer bis zu 35, z.B. Chlor oder Brom, und/oder Nitro, substituiertes 2-Pyrazinyl, sowie für gegebenenfalls entsprechend substituiertes Pyridyl, z.B. 2- oder 3-Pyridyl steht, wobei Substituenten irgendeine Stellung, mindestens einer jedoch vorzugsweise die ortho-Stellung zum Verknüpfungsringkohlenstoffatom und/oder Stickstoffatom des Heteroarylrestes einnehmen können, sowie 4-Indolyl, und $R_2$ Wasserstoff, Niederalkyl, z.B. Methyl, Aethyl oder Isopropyl, oder Phenylniederalkyl, z.B. Benzyl oder 1- oder 2-Phenyläthyl, bedeutet, und alk Niederalkylen mit 2-3 Kohlenstoffatomen bedeutet, das die beiden Stickstoffatome durch 2-3 Kohlenstoffatome trennt, z.B. Aethylen oder 1,3-Propylen, und Salze, insbesondere Säureadditionssalze, in erster Linie pharmazeutisch verwendbare, nicht-toxische Säureadditionssalze davon.

Die Erfindung betrifft in erster Linie Verbindungen der Formel I, worin $R_1$ für 2-Pyrazinyl, ferner für Pyridyl, z.B. 2- oder 3-Pyridyl, sowie 4-Indolyl steht, das in ortho-Stellung zum Verknüpfungskohlenstoffatom und/oder Stickstoffatom vorzugsweise durch Niederalkyl, z.B. Methyl, Niederalkoxy, z.B. Methoxy, Niederalkylthio, z.B. Methylthio oder Aethylthio, Halogen mit Atomnummer bis zu 35, z.B. Chlor oder Brom, oder Nitro substituiert ist, und gegebenenfalls weitere Substituenten dieser Art enthalten kann, $R_2$ insbesondere für Wasserstoff, ferner für Niederalkyl, z.B. Methyl, oder Phenylniederalkyl, z.B. Benzyl steht, und alk Aethylen darstellt, und Salze, insbesondere Säureadditionssalze, in erster Linie pharmazeutisch verwendbare, nicht-toxische Säureadditionssalze davon.

Die Erfindung betrifft in erster Linie Verbindungen der Formel I, worin $R_1$ für 4-Indolyl, 2-Pyrazinyl oder Pyridyl steht, das in ortho-Stellung zum Verknüpfungskohlenstoffatom und/oder Stickstoffatom vorzugsweise durch Niederalkyl, z.B. Methyl, Niederalkoxy, z.B. Methoxy, Niederalkylthio, z.B. Methylthio oder Aethylthio, Halogen mit Atomnummer bis zu 35, z.B. Chlor oder Brom, oder Nitro substituiert ist, $R_2$ Wasserstoff, ferner Niederalkyl, z.B. Methyl, bedeutet, und alk Aethylen darstellt, und Salze, insbesondere Säureadditionssalze, in erster Linie pharmazeutisch verwenbdare, nicht-toxische Säureadditionssalze davon.

Die Erfindung betrifft in erster Linie die in den Beispielen beschriebenen Verbindungen, insbesondere diejenigen der Formel I, die als Rest $R_1$ einen vorzugsweise substituierten 2-Pyrazinylrest aufweisen.

Die neuen Verbindungen werden nach an sich bekannten Methoden erhalten. So kann man z.B. eine Verbindung der Formel

$$R_1 - O - X_1 \qquad (II)$$

oder ein Salz davon mit einer Verbindung der Formel

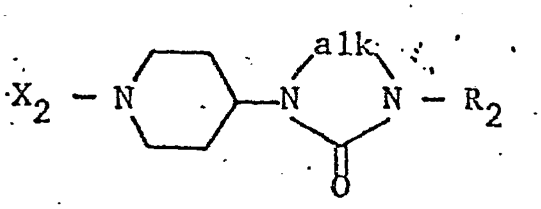

(III)

oder einem Salz davon umsetzen, worin einer der Reste $X_1$ und $X_2$ Wasserstoff bedeutet, und der andere dem Rest der Formel

$$-CH_2-\underset{}{\overset{X_3}{CH}}-CH_2-X_4 \qquad (IV)$$

entspricht, worin $X_3$ für eine freie Hydroxylgruppe steht, und $X_4$ eine reaktionsfähige veresterte Hydroxylgruppe be-

deutet, oder worin $X_3$ und $X_4$ zusammen eine Epoxygruppe bilden, und, wenn erwünscht, eine erhältliche Verbindung in eine andere Verbindung der Formel I umwandelt, und/oder, wenn erwünscht, eine erhältliche freie Verbindung in ein Salz überführt, und/oder, wenn erwünscht, ein erhältliches Salz in die freie Verbindung oder in ein anderes Salz überführt, und/oder, wenn erwünscht, ein erhältliches Isomerengemisch in die Isomeren auftrennt.

So kann man bei der obigen Reaktion z.B. so vorgehen, dass man eine Verbindung der Formel

$$R_1 - O - CH_2 - \overset{X_3}{\overset{|}{CH}} - CH_2 - X_4 \qquad (IIa)$$

mit einer Verbindung der Formel

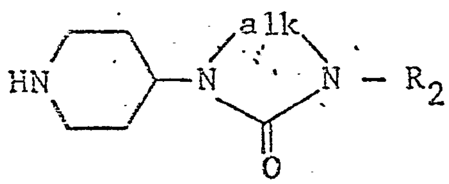

(IIIa),

oder einem Salz davon, oder dass man eine Verbindung der Formel $R_1 - OH$ (IIIb) oder ein Salz davon mit einer Verbindung der Formel

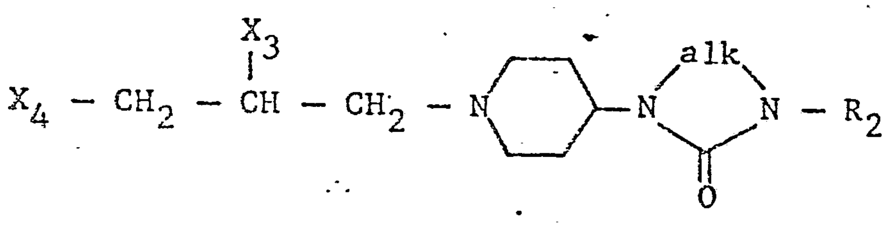

(IIb)

umsetzt, worin entweder $X_3$ für freies Hydroxy und $X_4$ für eine reaktionsfähige veresterte Hydroxylgruppe stehen oder $X_3$ und $X_4$ zusammen eine Epoxygruppe bilden.

Eine reaktionsfähige veresterte Hydroxylgruppe $X_4$ ist eine, durch eine starke, anorganische oder organische Säure, vor allem eine Halogenwasserstoffsäure,

BAD ORIGINAL

z.B. Chlorwasserstoffsäure, Bromwasserstoffsäure oder Jodwasserstoffsäure, ferner Schwefelsäure oder durch eine organische Sulfonsäure, wie eine aromatische Sulfonsäure, z.B. Benzolsulfonsäure, 4-Brombenzolsulfonsäure oder 4-Toluolsulfonsäure, veresterte Hydroxylgruppe. So steht $X_4$ insbesondere für Chlor oder Brom, ferner Jod, falls es nicht zusammen mit $X_3$ eine Epoxy-Gruppierung bildet.

Die obige Umsetzung wird in üblicher Weise durchgeführt. Bei Verwendung eines reaktionsfähigen Esters als Ausgangsmaterial der Formel IIa wird vorzugsweise in Gegenwart eines basischen Kondensationsmittels und/oder mit einem Ueberschuss der basischen Verbindung der Formel IIIa gearbeitet.

Falls man einen reaktionsfähigen Ester der Formel IIb als Ausgangsmaterial verwendet, wird die Verbindung der Formel IIIb vorzugsweise in Form eines Salzes, wie eines Metall-, insbesondere Alkalimetall-, z.B. Natrium- oder Kaliumsalzes, eingesetzt, oder man arbeitet in Gegenwart eines säurebindenden Mittels, insbesondere eines Kondensationsmittels, welches mit der Verbindung der Formel IIIb ein Salz zu bilden vermag, wie einem Alkalimetall-niederalkoxid.

Die obige Reaktion wird in Abwesenheit oder vorzugsweise in Anwesenheit eines, üblicherweise inerten, Lösungs- oder Verdünnungsmittels, und, wenn notwendig, unter Kühlen oder Erwärmen, z.B. in einem Temperaturbereich von etwa 0°C bis etwa 150°C, in einem geschlossenen Gefäss und/oder in einer Inertgas-, z.B. Stickstoffatmosphäre durchgeführt.

Die Ausgangsstoffe sind bekannt oder können in an sich bekannter Weise erhalten werden. So kann man z.B. Piperidin-4-on, worin die sekundäre Aminogruppe gegebenenfalls in an sich bekannter Weise, z.B. durch einen Benzyl-

rest oder einen leicht abspaltbaren Acylrest geschützt sein kann, mit einem Diamin der Formel $H_2N$-alk-HN-$R_2$ (V) umsetzen und gleichzeitig oder nachträglich mit einem geeigneten Reduktionsmittel, wie katalytisch aktiviertem Wasserstoff oder einem Hydridreduktionsmittel, z.B. Natriumcyanborhydrid, behandeln. Im so erhältlichen Zwischenprodukt bildet man den 2-Oxo-1,3-diazacycloalkanrest, z.B. durch Behandeln mit einem geeigneten reaktionsfähigen Kohlensäurederivat, wie Diniederalkylcarbonat oder Phosgen; falls notwendig, kann eine N-Schutzgruppe in an sich bekannter Weise durch Wasserstoff ersetzt werden. Ein so erhältliches Ausgangsmaterial der Formel IIIa kann in an sich bekannter Weise, z.B. durch Behandeln mit einem reaktionsfähigen Ester eines 2,3-Epoxy-1-propanols, wie einem 2,3-Epoxy-1-propylhalogenid, und, falls erwünscht, durch nachträgliches Umsetzen mit einer starken Säure, wie einer Halogenwasserstoffsäure, in ein Ausgangsmaterial der Formel IIb umgewandelt werden.

Die neuen Verbindungen können ebenfalls erhalten werden, indem man in einer Verbindung der Formel

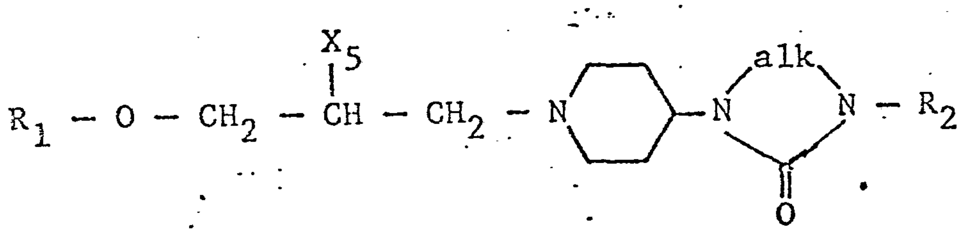 (VI)

worin $X_5$ einen in Hydroxy überführbaren Rest darstellt, $X_5$ in Hydroxy überführt, und, wenn erwünscht, die zusätzlichen Verfahrensschritte durchführt.

In einem Ausgangsmaterial der Formel VI ist $X_5$ insbesondere eine veresterte Hydroxygruppe und in erster Linie Acyloxy, worin Acyl den entsprechenden Rest einer organischen Carbonsäure, wie einer Niederalkancarbonsäure,

BAD ORIGINAL

z.B. Acetyl, Propionyl oder Pivaloyl, oder einer aromatischen Carbonsäure, z.B. Benzoyl, darstellt.

Die Ueberführung von $X_5$ in Hydroxy kann mittels Solvolyse vorgenommen werden, insbesondere durch Hydrolyse, wobei man mit Wasser in einem alkalischen oder sauren Medium arbeiten kann, oder durch Alkoholyse (Umesterung), wobei man z.B. mit einem Niederalkanol, z.B. Methanol oder Aethanol, gegebenenfalls in Gegenwart eines Umesterungskatalysators, wie einem Alkalimetallniederalkanolat, z.B. Natrium- oder Kaliummethanolat, -äthanolat oder -butanolat, umsetzt. Die Reaktion wird in Ab- oder Anwesenheit von Lösungs- oder Verdünnungsmitteln, und, wenn notwendig, unter Kühlen oder Erwärmen, z.B. in einem Temperaturbereich von etwa 0°C bis etwa 120°C, in einem geschlossenen Gefäss und/oder in einer Inertgasatmosphäre durchgeführt.

Die Ausgangsstoffe der Formel VI können z.B. erhalten werden, indem man in einer Verbindung der Formel IIa, worin $X_3$ für Hydroxy steht und $X_4$ eine reaktionsfähige veresterte Hydroxygruppe bedeutet, $X_3$ in eine, in Hydroxy überführbare veresterte Hydroxygruppe, z.B. durch Acylieren mit einem reaktionsfähigen Derivat, wie einem gegebenenfalls gemischten Anhydrid, einer organischen Carbonsäure, in Acyloxy überführt und das so erhältliche Zwischenprodukt, vorzugsweise im Ueberschuss, mit einer Verbindung der Formel IIIa umsetzt.

Die neuen Verbindungen können ebenfalls erhalten werden, wenn man in einer Verbindung der Formel

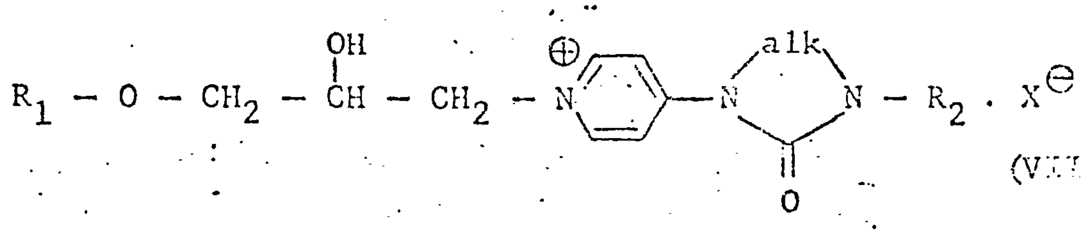

(VII)

worin $X^{\ominus}$ ein Anion bedeutet, den Pyridiniumring zum Piperidinring reduziert, und, wenn erwünscht, die zusätzlichen Verfahrensschritte durchführt.

Ein Anion $X^{\ominus}$ ist insbesondere dasjenige einer Säure, vorzugsweise einer Mineral-, wie Halogenwasserstoff-, z.B. Chlor- oder Bromwasserstoffsäure, oder einer geeigneten organischen Carbon- oder Sulfonsäure.

Die obige Reduktion kann in üblicher Weise, vorzugsweise mittels katalytischer Hydrierung, wie mit Wasserstoff in Gegenwart eines geeigneten Hydrierungskatalysators, z.B. eines Schwermetall-, z.B. Palladium-, Platin- oder Raney-Nickel-Katalysators, oder durch Behandeln mit nascierendem Wasserstoff, wie durch Behandeln mit einem Alkalimetall, z.B. Natrium oder Kalium, in Gegenwart eines Alkohols, wie Niederalkanol, z.B. Aethanol oder n-Butanol, durchgeführt werden.

Dabei kann die Reduktion, bei welcher darauf geachtet werden muss, dass andere reduzierbare Gruppen nicht angegriffen werden, auch stufenweise erfolgen, da als Zwischenprodukte teilweise gesättigte Pyridin-, z.B. 1,2,5,6-Tetrahydro-pyridinverbindungen gebildet werden können, die beim Behandeln mit dem gleichen Reduktionsmittel, gegebenenfalls unter anderen Bedingungen, oder mit einem anderen Reduktionsmittel in die gewünschten Piperidinverbindungen übergeführt werden können.

Die obige Reaktion wird in Abwesenheit und insbesondere in Anwesenheit eines Lösungs- oder Verdünnungsmittels und, wenn notwendig, unter Kühlen oder Erwärmen, z.B. in einem Temperaturbereich von etwa 0°C bis etwa 120°C, in einem geschlossenen Gefäss und/oder in einer Inertgasatmosphäre durchgeführt.

BAD ORIGINAL

Die Ausgangsstoffe können in an sich bekannter Weise hergestellt werden, indem man z.B. 4-Amino-pyridin mit einer Verbindung der Formel $Hal_1$-$alk_o$-C(=O)-NH-$R_2$ (VIII) umsetzt, wobei in letzterer $alk_o$ dem um ein Kettenkohlenstoffatom verkürzten Niederalkylenrest entspricht; man erhält diese z.B. durch Behandeln einer Aminoverbindung der Formel $H_2N$-$R_2$ (IX) mit einem Säurehalogenid der Formel $Hal_1$-$alk_o$-C(=O)-$Hal_2$ (X), worin $Hal_1$ und $Hal_2$ je für Halogen, z.B. Chlor, stehen. Im so gebildeten Zwischenprodukt der Formel

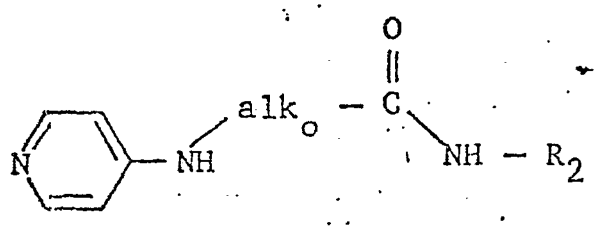

(XI)

wird die Carbonylgruppe, z.B. durch Behandeln mit Lithiumaluminiumhydrid, zur Methylengruppe reduziert und der 2-Oxo-1,3-diazacycloalkanring, z.B. durch Behandeln mit Phosgen, gebildet. Die so erhältliche 4-(2-Oxo-3-$R_2$-1,3-diazacycloalkyl-1-yl)-pyridinverbindung wird dann mit einer Verbindung der Formel IIa, worin $X_3$ für Hydroxy und $X_4$ für eine reaktionsfähige veresterte Hydroxygruppe, insbesondere Halogen, stehen, umgesetzt.

Die neuen Verbindungen der Formel I können ebenfalls erhalten werden, indem man in einer Verbindung der Formel

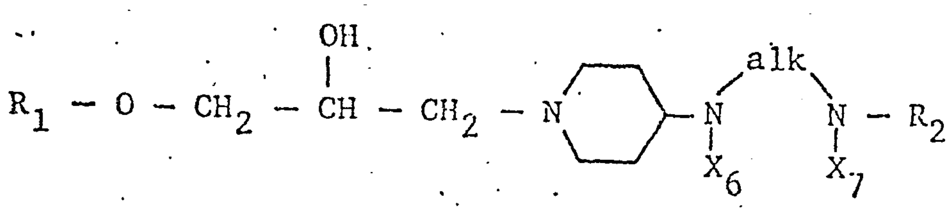

(XII)

worin einer der Reste $X_6$ und $X_7$ für Wasserstoff steht und der andere den Acylrest eines Kohlensäurehalbderivats bedeutet, den 2-Oxo-1,3-diazacycloalkanring durch Ringschluss bildet, und, wenn erwünscht, die zusätzlichen Verfahrensstufen durchführt.

In einem Ausgangsmaterial der Formel XII bedeutet der Acylrest eines Kohlensäurehalbderivats den entsprechenden Rest eines Kohlensäurehalbesters, wie Niederalkoxycarbonyl, z.B. Methoxycarbonyl oder Aethoxycarbonyl, eines Kohlensäurehalbhalogenids, d.h. Halogencarbonyl, z.B. Chlorcarbonyl oder Bromcarbonyl, oder eines Kohlensäurehalbamids, z.B. Carbamoyl.

Der Ringschluss wird in Ab- oder Anwesenheit eines Lösungs- oder Verdünnungsmittels, und, wenn notwendig, in Gegenwart eines, vorzugsweise basischen Kondensationsmittels, wie eines Alkalimetall- oder Erdalkalimetall-hydroxids, -carbonats, -hydrogencarbonats oder -niederalkanoats, sowie einer organischen Base, wie eines tert.-Amins oder einer Base vom Pyridintyp, unter Kühlen oder vorzugsweise Erwärmen, z.B. in einem Temperaturbereich von etwa +20°C bis etwa 150°C, in einem geschlossenen Gefäss und/oder in einer Inertgasatmosphäre durchgeführt.

Das Ausgangsmaterial der Formel XII wird nach an sich bekannten Methoden und vorzugsweise <u>in situ</u> hergestellt, indem man eine Verbindung der Formel XII, worin $X_6$ und $X_7$ für Wasserstoff stehen, mit einem reaktionsfähigen Derivat der Kohlensäure umsetzt. Reaktionsfähige Derivate der Kohlensäure sind entsprechende Ester, wie Diniederalkylcarbonat, z.B. Diäthylcarbonat, oder Halogenide, z.B. Phosgen, ferner Amide, z.B. Harnstoff oder Carbonyldiimidazol, sowie auch Halogenkohlensäureester, wie Chlorkohlensäure-niederalkylester, z.B. Chlorkohlensäureisobutylester, oder Carbaminsäurehalogenide, z.B. -chlorid.

Die Umsetzung wird in üblicher Weise durchgeführt, normalerweise in Gegenwart eines inerten Lösungsmittels, vorzugsweise eines gegebenenfalls halogenierten aliphatischen oder aromatischen Kohlenwasserstoffs, z.B. Chloroform oder Toluol, ferner eines Amids oder Nitrils, z.B. Dimethylformamid, Dimethylacetamid oder Acetonitril, oder eines cycloaliphatischen Aethers, wie Dioxan und Tetrahydrofuran. Die Reaktion wird vorzugsweise in Gegenwart eines Kondensationsmittels, besonders eines basischen Kondensationsmittels, wie eines Alkalimetall- oder Erdalkalimetallhydroxids, -carbonat oder -hydrogencarbonats, z.B. Natrium- oder Kaliumhydroxid, Natrium- oder Kaliumcarbonat oder -hydrogencarbonat, oder eines Alkalimetallniederalkanoats, z.B. Natriumacetat, oder eines Alkalimetallniederalkanolats, z.B. Natriummethanolat oder Kalium-tert.-butanolat, oder einer organischen tertiären Stickstoffbase, wie eines Triniederalkylamins, z.B. Trimethylamin oder Triäthylamin, oder Pyridin, vorgenommen.

Eine Verbindung der Formel XII, worin $X_6$ und $X_7$ für Wasserstoff stehen, kann man z.B. erhalten, indem man Piperidin-4-on mit einer Verbindung der Formel IIa, worin $X_3$ Hydroxy bedeutet, und $X_4$ für eine reaktionsfähige veresterte Hydroxygruppe, insbesondere Halogen steht, umsetzt, und anschliessend das Zwischenprodukt mit einem Diamin der Formel V unter gleichzeitiger oder nachträglicher Behandlung mit einem Reduktionsmittel, wie katalytisch aktiviertem Wasserstoff oder einem geeigneten Hydridreduktionsmittel, z.B. Natriumcyanborhydrid, reagieren lässt.

Die neuen Verbindungen der Formel I können ebenfalls erhalten werden, wenn man in einer Verbindung der Formel

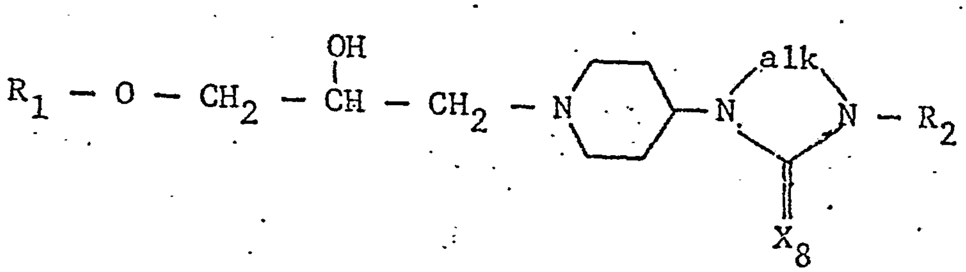 (XIII)

worin $X_8$ einen in Oxo überführbaren Iminorest darstellt, $X_8$ in Oxo überführt, und, wenn erwünscht, die zusätzlichen Verfahrensschritte durchführt.

Der Iminorest $X_8$ kann, z.B. durch Niederalkyl oder Phenyl, substituiert sein. Ein entsprechendes Ausgangsmaterial der Formel XIII kann man durch Hydrolyse, vorzugsweise in Gegenwart eines sauren Mittels, wie einer Mineralsäure, z.B. Chlorwasserstoffsäure, in die entsprechende Verbindung der Formel I überführen.

Die obige Reaktion wird in Ab- oder vorzugsweise in Anwesenheit eines Lösungs- oder Verdünnungsmittels und, wenn notwendig, unter Kühlen oder Erwärmen, z.B. in einem Temperaturbereich von etwa 0°C bis etwa 150°C, gegebenenfalls in einem geschlossenen Gefäss und/oder in einer Inertgasatmosphäre durchgeführt.

Die Ausgangsstoffe der Formel XIII können in an sich bekannter Weise erhalten werden, z.B. durch Behandeln einer Verbindung der Formel XII, worin jeder der Reste $X_6$ und $X_7$ für Wasserstoff steht, mit einem Halogencyan, z.B. Bromcyan, vorzugsweise in Gegenwart eines geeigneten, z.B. basischen Kondensationsmittels, unter gleichzeitigem oder nachträglichem Ringschluss zum 2-Imino-1,3-diazacycloalkanring des Ausgangsmaterials der Formel XIII.

In erhaltenen Verbindungen kann man im Rahmen der Endstoffe in an sich bekannter Weise Substituenten abspalten, einführen oder umwandeln.

So kann man in Verbindungen der Formel I mit ungesättigten Substituenten, z.B. Niederalkenyl, Niederalkenyloxy oder Niederalkinyloxy, diese durch geeignete Reduktionsmethoden zu entsprechenden gesättigten Verbindungen oder, im Fall von Substituenten mit einer Dreifachbindung, zu Verbindung mit einer Doppelbindung reduzieren. Dabei verwendet man als Reduktionsmittel vorzugsweise katalytisch aktivierten Wasserstoff. Als Katalysator eignet sich insbesonder der Lindlar-Katalysator (Pd-Pb-$CaCO_3$).

Man kann auch in einer erhaltenen Verbindung der Formel I, die als Substituenten eines aromatischen Restes Halogen, wie Brom oder Jod, enthält, dieses z.B. durch Behandeln mit Trifluormethyljodid in Gegenwart von Kupferpulver und eines geeigneten aprotischen Lösungsmittels, wie Pyridin, Dimethylformamid oder Acetonitril, durch Trifluormethyl ersetzen.

In einer erhaltenen Verbindung der Formel I kann man eine α-Phenylniederalkylgruppe, z.B. in Benzyloxy, durch Behandeln der entsprechenden Verbindung mit katalytisch aktiviertem Wasserstoff abspalten und durch Wasserstoff, z.B. eine Benzyloxygruppe durch Hydroxy, ersetzen.

Ferner kann man in einer Verbindung der Formel I, die Hydroxy oder Mercapto in der Form einer primären Carbinol- oder einer phenolischen Hydroxylgruppe als Substituenten enthält, dieses, gegebenenfalls in Salz-, z.B. Alkalimetallsalzform, durch Behandeln mit einem reaktionsfähigen Ester eines Alkohols, wie einem gegebenenfalls substituierten Niederalkylhalogenid, in veräthertes

Hydroxy oder Mercapto, z.B. Niederalkoxy bzw. Niederalkylthio, umwandeln. Zudem kann man Hydroxy in einem Hydroxyniederalkyl- oder Hydroxyniederalkoxy-Substituenten, üblicherweise in Form einer reaktionsfähigen veresterten Hydroxygruppe, wie Halogen, z.B. Chlor, mit einem Alkohol, z.B. Niederalkanol, oder einem Mercaptan, z.B. Niederalkylmercaptan, vorzugsweise in Gegenwart eines basischen Mittels, das z.B. einen Alkohol oder ein Mercaptan in eine Metallverbindung überzuführen vermag, umsetzen und so zu Verbindungen der Formel I gelangen, die entsprechend veräthertes Hydroxy- oder Mercapto-niederalkyl bzw. -niederalkoxy aufweisen. Ferner kann man in einer erhaltenen Verbindung eine reaktionsfähige veresterte Hydroxygruppe, wie Halogen, z.B. Chlor, insbesondere in α-Stellung zu einem Ringstickstoffatom in einem Rest $R_1$, z.B. durch Behandeln mit einer Alkoholat- oder Thiolatverbindung, wie einem Alkalimetall-, z.B. Natrium- oder Kalium-niederalkanolat oder -thioniederalkanolat, in eine verätherte oder veresterte Hydroxy- oder Mercaptogruppe, z.B. in Niederalkoxy oder Niederalkylthio, umwandeln.

In einer Verbindung der Formel I kann man eine Propargyloxygruppe, z.B. durch Hydratisierung in saurem Medium und in Gegenwart eines Quecksilber-II-salzes, z.B. durch Behandeln mit einer wässerigen Mineralsäure, z.B. verdünnter Salz- oder Schwefelsäure, in Gegenwart von Quecksilber-II-chlorid, in die Acetonyloxygruppe umwandeln.

Ferner kann man in einer Verbindung der Formel I, die als Substituenten verestertes Carboxyl oder Niederalkoxycarbonylamino enthält, dieses, z.B. durch Behandeln mit Ammoniak oder einem Amin, vorzugsweise mit einem Ueberschuss davon und bei erhöhter Temperatur, in

BAD ORIGINAL

amidiertes Carboxyl bzw. gegebenenfalls Ureido überführen.

Man kann auch in einer Verbindung der Formel I, die primäres Amino als Substituenten enthält, dieses substituieren; so kann man Amino, z.B. durch Behandeln der Aminoverbindung mit einem geeigneten Säurederivat, wie einem gegebenenfalls gemischten Anhydrid, z.B. einem entsprechenden Chlorid, falls notwendig in Gegenwart eines basischen Mittels, acylieren.

Die oben beschriebenen Reaktionen können gegebenenfalls gleichzeitig oder nacheinander und in beliebiger Reihenfolge und in üblicher Weise, z.B. in An- oder Abwesenheit von Lösungs- oder Verdünnungsmitteln, falls notwendig, in Gegenwart von Kondensations- und/oder katalytischen Mitteln, unter Kühlen oder Erwärmen, in einem geschlossenen Gefäss und/oder in einer Inertgasatmosphäre durchgeführt werden.

Je nach den Verfahrensbedingungen und Ausgangsstoffen erhält man die Endstoffe in freier Form oder in der ebenfalls von der Erfindung umfassten Form ihrer Salze, insbesondere ihrer Säureadditionssalze. Erhaltene Salze können in an sich bekannter Weise in die freien Verbindungen übergeführt werden, Säureadditionssalze z.B. durch Behandeln mit basischen Mitteln, inkl. geeigneten Ionenaustauschern. Andererseits können erhaltene freie Verbindungen, z.B. durch Behandeln mit organischen oder anorganischen Säuren, Salze bilden. Ferner kann man erhaltene Salze, Säureadditionssalze z.B. durch Behandeln mit geeigneten Schwermetallsalzen oder Anionenaustauschern, in andere Salze überführt.

Die oben genannten Salze oder andere Salze der neuen Verbindungen der Formel I, wie z.B. die Pikrate, können auch zur Reinigung der erhaltenen, freien Basen

BAD ORIGINAL

dienen, indem man die freien Basen in Salze überführt, diese abtrennt und aus den Salzen wiederum die Basen freimacht. Infolge der engen Beziehungen zwischen den neuen Verbindungen in freier Form und in Form ihrer Salze sind im Vorausgegangenen und nachfolgend unter den freien Verbindungen sinn- und zweckmässig gegebenenfalls auch die entsprechenden Salze zu verstehen.

Die neuen Verbindungen können je nach der Wahl der Ausgangsstoffe und Arbeitsweisen in Form der Racemate oder der optischen Antipoden vorliegen.

Erhaltene Racemate lassen sich nach bekannten Methoden in die optischen Antipoden auftrennen, beispielsweise durch Umkristallisieren aus einem optisch aktiven Lösungsmittel, mit Hilfe von Mikroorganismen oder durch Umsetzen mit einer, mit der racemischen Verbindung Salze bildenden optisch aktiven Säure und Trennen der auf diese Weise erhaltenen Salze, z.B. aufgrund ihrer verschiedenen Löslichkeiten, in die diastereomeren Salze, aus denen die freien Antipoden durch Einwirkung geeigneter Mittel freisetzt werden können. Besonders geeignete, optisch aktive Säuren sind z.B. die D- und L-Formen von Weinsäure, Di-Toluylweinsäure, Aepfelsäure, Mandelsäure, Camphersulfonsäure oder Chinasäure.

Die Erfindung betrifft auch diejenigen Ausführungsformen des Verfahrens, nach denen man von einer, auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form eines Derivates, z.B. eines Salzes vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die neuen Verbindungen können z.B. in Form pharmazeutischer Präparate Verwendung finden, welche eine pharmakologisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und/oder Schmiermitteln, z.B. Kieselerde, Talk, Stearinsäure oder Salze davon, wie Magnesium- oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel aufweisen. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermitteler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 5 mg und etwa 50 mg für Warmblüter mit einem Gewicht von etwa 70 kg.

Die folgenden Beispiele dienen zur Illustration der Erfindung; Temperaturen werden in Celsiusgraden angegeben.

## Beispiel 1

Ein Gemisch von 22,5 g rohem 3-(2,3-Epoxy-propyloxy)-2-methoxy-pyridin und 21,0 g 1-(4-Piperidyl)-imidazolidin-2-on in 250 ml Isopropanol wird 14 Stunden unter Rückfluss gekocht. Das Reaktionsgemisch wird eingedampft, der Rückstand in Essigsäureäthylester gelöst und mit 2-n. Salzsäure extrahiert. Der saure Extrakt wird mit einer konzentrierten wässerigen Natriumhydroxidlösung alkalisch gestellt und mit Essigsäureäthylester extrahiert. Die organische Lösung wird eingedampft und der Rückstand aus einem Gemisch von Methylenchlorid und Diäthyläther umkristallisiert. Das so erhaltene 1-{1-[2-Hydroxy-3-(2-methoxy-3-pyridyloxy)-propyl]-4-piperidyl}-imidazolidin-2-on schmilzt bei 132-134°.

Das Ausgangsmaterial kann wie folgt erhalten werden:

Ein Gemisch von 15,4 g 3-Hydroxy-2-methoxy-pyridin, 16 g Kaliumcarbonat, 25 ml Epichlorhydrin und 150 ml Acetonitril wird 7 Stunden unter Rückfluss gekocht. Durch Abkühlen, Filtrieren und Eindampfen des Filtrats erhält man das rohe 3-(2,3-Epoxy-propyloxy)-2-methoxy-pyridin, welches ohne weitere Reinigung verwendet wird.

In analoger Weise kann man durch Umsetzen von 20 g rohem 3-(2,3-Epoxy-propyloxy)-2-methoxy-6-methyl-pyridin und 16,5 g 1-(4-Piperidyl)-imidazolidin-2-on das 1-{1-[2-Hydroxy-3-(2-methoxy-6-methyl-3-pyridyloxy)-propyl]-4-piperidyl}-imidazolidin-2-on erhalten, das nach Umkristallisieren aus einem Gemisch von Methylenchlorid und Diäthyläther bei 144-147° schmilzt. Das Ausgangsmaterial kann analog dem oben beschriebenen Verfahren aus 3-Hydroxy-2-methoxy-6-methyl-pyridin erhalten werden und

wird ohne weitere Reinigung umgesetzt.

Beispiel 2

Eine Lösung von 25,0 g 2-(2,3-Epoxy-propyloxy)-3-methoxy-pyridin und 23,3 g 1-(4-Piperidyl)-imidazolidin-2-on in 500 ml Isopropanol wird 24 Stunden bei 30° gerührt. Durch Aufarbeiten analog dem im Beispiel 1 beschriebenen Verfahren erhält man nach Versetzen mit Aceton das 1-{1-[2-Hydroxy-3-(3-methoxy-2-pyridyloxy)-propyl]-4-piperidyl}-imidazolidin-2-on, F. 137-142°. Die Verbindung bildet ein neutrales Fumarat, das nach Umkristallisieren aus einem Gemisch aus Isopropanol und Aceton als Hydrat bei 124-126° schmilzt.

Das Ausgangsmaterial kann auf folgende Weise hergestellt werden:

Ein Gemisch von 161 g 3-Methoxy-2-nitro-pyridin und 144 g 2,2-Dimethyl-5-hydroxymethyl-1,3-dioxolan in 1000 ml Hexamethylphosphorsäuretriamid wird unter Rühren im Verlauf einer Stunde mit 26,5 g Natriumhydrid versetzt; durch Kühlen wird die Temperatur während der Zugabe bei 0-10° gehalten. Das Reaktionsgemisch wird während weiteren 5 Stunden unter Eiskühlung und dann 15 Stunden bei Ramtemperatur gerührt. Das Reaktionsgemisch wird auf Eis gegossen und mit Diäthyläther extrahiert. Der organische Extrakt wird mit einer konzentrierten wässerigen Natriumchloridlösung gewaschen, getrocknet und eingedampft. Der Rückstand wird in 1000 ml Aethanol gelöst, mit 100 ml 2-n. Salzsäure versetzt und 8 Stunden stehen gelassen. Nach dem Abdampfen des Lösungsmittels wird der Rückstand mit einer konzentrierten Lösung von Natriumhydroxid in Wasser alkalisch gestellt und mit Essigsäureäthylester extrahiert. Durch Abdampfen

des Lösungsmittels erhält man ein Rohprodukt, aus dem nach Zugabe von Diäthyläther das kristalline 3-(3-Methoxy-2-pyridyloxy)-1,2-propandiol, F. 62-65°, erhalten wird.

Eine Lösung von 62 g 3-(3-Methoxy-2-pyridyloxy)-1,2-propandiol in 350 ml Orthoessigsäure-triäthylester wird mit 2 Tropfen Trifluoressigsäure versetzt und 3 Stunden bei 20-30° stehen gelassen. Durch Eindampfen erhält man das rohe 2-Aethoxy-5-(3-methoxy-2-pyridyloxymethyl)-2-methyl-1,3-dioxolan als Oel, das ohne Reiniverwendet wird.

Ein Gemisch von 85 g 2-Aethoxy-5-(3-methoxy-2-pyridyloxymethyl)-2-methyl-1,3-dioxolan in 500 ml Dichlormethan wird mit 45 ml Trimethylchlorsilan versetzt und während einer Stunde bei 20-30° gerührt. Durch vollständiges Eindampfen unter vermindertem Druck erhält man das rohe 2-(2-Acetyloxy-3-chlor-propyloxy)-3-methoxypyridin als Oel, welches ohne Reinigung verwendet wird.

Ein Gemisch von 80 g 2-(2-Acetyloxy-3-chlorpropoxy)-3-methoxy-pyridin, 900 ml Methylenchlorid, 500 ml einer 2-n. wässerigen Natriumhydroxidlösung und 9,5 g Tetrabutylammoniumhydrogensulfat wird 16 Stunden bei 20-30° kräftig gerührt. Die organische Phase wird dann abgetrennt und eingedampft. Das verbleibende Oel wird in Diäthyläther gelöst; die Lösung wird filtriert, mit Aktivkohle behandelt und eingedampft. Man erhält so das 2-(2,3-Epoxy-propyloxy)-3-methoxy-pyridin, F. 63-65°.

Beispiel 3

Ein Gemisch von 14,9 g 2-Chlor-3-(2,3-epoxy-propyloxy)-pyrazin (siehe z.B. deutsche Offenlegungsschrift 25 20 910) und 10,4 g 1-(4-Piperidyl)-imidazolidin-2-on in 150 ml Isopropanol wird 24 Stunden bei etwa 20° gerührt. Aus dem Reaktionsgemisch beginnt ein kristalliner Niederschlag auszufallen; zur vollständigen Kristallisation werden noch 150 ml Diäthyläther zugegeben. Man erhält so das 1-{1-[3-(3-Chlor-2-pyrazinyloxy)-2-hydroxy-propyl]-4-piperidyl}-imidazolidin-2-on, F. 150 - 151°. Das mit der berechneten Menge Fumarsäure hergestellte neutrale Fumarat kristallisiert aus einem Gemisch von Methanol und Diäthyläther und schmilzt bei 172-173°.

Beispiel 4

Ein Gemisch von 10,66 g 1-{1-[3-(3-Chlor-2-pyrazinyloxy)-2-hydroxy-propyl]-4-piperidyl}-imidazolidin-2-on und 1,78 g Natriummethylat in 150 ml Methanol wird während 10 Stunden unter Rühren am Rückfluss gekocht. Das Reaktionsgemisch wird im Wasserstrahlvakuum total eingedampft. Der Rückstand wird in Essigsäureäthylester gelöst und mit Wasser gewaschen. Die organische Lösung wird über Natriumsulfat getrocknet und im Wasserstrahlvakuum eingedampft. Man erhält so das 1-{1-[2-Hydroxy-3-(3-methoxy-2-pyrazinyloxy)-propyl]-4-piperidyl}-imidazolidin-2-on als Oel. Das mit einer methanolischen Chlorwasserstoffsäure hergestellte Hydrochlorid kristallisiert aus einem Gemisch von Methanol und Diäthyläther und schmilzt bei 222° - 223° (mit Zersetzen).

BAD ORIGINAL

Beispiel 5

Ein Gemisch von 8,65 g 2-Methyl-3-(2,3-epoxy-propyloxy)-pyrazin (siehe z.B. deutsche Offenlegungsschrift 25 20 910) und 6,75 g 1-(4-Piperidyl)-imidazolidin-2-on in 100 ml Isopropanol wird 60 Stunden bei etwa 20° gerührt. Das Reaktionsgemisch wird im Wasserstrahlvakuum total eingedampft, der Rückstand in Essigsäureäthylester gelöst und die Lösung mit 2-n. Salzsäure extrahiert. Die vereinigten salzsauren Extrakte werden mit einer konzentrierten wässerigen Natriumhydroxidlösung alkalisch gestellt und mit Methylenchlorid extrahiert. Die vereinigten organischen Extrakte werden mit wenig Wasser gewaschen, über Natriumsulfat getrocknet und im Wasserstrahlvakuum eingedampft. Man erhält so das ölige 1-{1-[2-Hydroxy-3-(3-methyl-2-pyrazinyloxy)-propyl]-4-piperidyl}-imidazolidin-2-on, das durch Behandeln mit der berechneten Menge Fumarsäure in das neutrale Fumarat übergeführt wird, F. 168°-170° nach Umkristallisieren aus einem Gemisch von Methanol und Aceton.

Beispiel 6

Ein Gemisch von 46,0 g 3-(2,3-Epoxy-propyloxy)-2-nitropyridin und 35,0 g 1-(4-Piperidyl)-imidazolidin-2-on in 500 ml Isopropanol wird 2 Stunden unter Rückfluss gekocht. Die Aufarbeitung, analog Beispiel 1 unter Verwendung von Dichlormethan als Extraktionsmittel, ergibt rohes 1-{1-[2-Hydroxy-3-(2-nitro-3-pyridyloxy)-propyl]-4-piperidyl}-imidazolidin-2-on, welches nach Umkristallisation aus Isopropanol bei 154-158° schmilzt.

Auf analoge Weise erhält man unter Verwendung von 13,0 g 3-(2,3-Epoxy-propyloxy)-2-nitropyridin und 11,0 g 3-Methyl-1-(4-piperidyl)-imidazolidin-2-on das 1-{1-[2-Hydroxy-3-(2-nitro-3-pyridyloxy)-propyl]-4-piperidyl}-3-methyl-imidazolidin-2-on als gelbes Oel.

Beispiel 7

Ein Gemisch von 7,3 g 1-Chlor-3-(2-chlor-3-pyridyloxy)-2-propanol und 5,0 g 3-Methyl-1-(4-piperidyl)-imidazolidin-2-on wird in 100 ml Isopropanol 2 Stunden unter Rückfluss gekocht. Die Aufarbeitung, analog Beispiel 1, ergibt öliges 1-{1-[2-Hydroxy-3-(2-chlor-3-pyridyloxy)-propyl]-4-piperidyl}-3-methyl-imidazolidin-2-on.

Auf analoge Weise erhält man unter Verwendung von 1-(4-Piperidyl)-imidazolidin-2-on das 1-{1-[2-Hydroxy 3-(2-chlor-3-pyridyloxy)-propyl]-4-piperidyl}-imidazolidin-2-on vom Smp. 163-166°.

Das Ausgangsmaterial wird auf folgende Weise hergestellt:

Ein Gemisch von 45 g 2-Chlor-3-pyridinol, 45 g Kaliumcarbonat, 150 ml Epichlorhydrin und 300 ml Aceton wird 5 Stunden unter Rückfluss gerührt. Durch Filtrieren, Eindampfen, Lösen in Aethylacetat und Waschen der Lösung mit Wasser erhält man nach dem Abdampfen des Aethylacetats rohes 1-Chlor-3-(2-chlor-3-pyridyloxy)-2-propanol als dunkelbraunes Oel, das durch Säulenchromatographie an Kieselgel und Elution mit Aether gereinigt wird und dann bei 140 - 143° schmilzt.

Beispiel 8

1,1 g 2-Chlor-3-(2,3-epoxy-propyloxy)-6-methyl-pyridin und 0,76 g 1-(4-Piperidyl)-imidazolidin-2-on werden in 40 ml Isopropanol 1 Stunde unter Rückfluss gekocht. Durch Abdampfen des Lösungsmittels und Umkristallisation aus Isopropanol-Aether erhält man 1-{1-[2-Hydroxy-3-(2-chlor-6-methyl-3-pyridyloxy)-propyl]-4-piperidyl}-imidazolidin-2-on vom Smp. 175 - 180°.

Das als Ausgangsmaterial verwendete 2-Chlor-3-(2,3-epoxy-propyloxy)-6-methyl-pyridin erhält man durch Kochen von 2-Chlor-6-methyl-3-pyridinol mit Epichlorhydrin und Kaliumcarbonat während 2 Stunden. Es wird roh weiterverwendet.

Beispiel 9

2,5 g 1-{1-[2-Hydroxy-3-(2-benzyloxy-3-pyridyloxy)-propyl]-4-piperidyl}-imidazolidin-2-on wird in 50 ml Methanol unter Zusatz von 0,3 g Pd/C 5% bis zum Stillstand bei Atmosphärendruck und 20-30° hydriert. Durch Abfiltrieren des Katalysators und Eindampfen der Lösung erhält man 3-{2-Hydroxy-3-[4-(imidazolidin-2-on-1-yl)-1-piperidyl]-propyloxy}-2-pyridin vom Smp. 217-222°.

Das Ausgangsmaterial wird auf folgende Weise erhalten:

a) 15,7 g 3-(2,3-Epoxy-propyloxy)-2-nitro-pyridin und 9,0 g Benzylalkohol, gelöst in 150 ml 1,2-Dimethoxyäthan werden unter Rühren und Kühlen bei 0-5° portionenweise mit 3,6 g einer Natriumhydrid-Suspension (55%) versetzt, 3 Stunden bei 0-5° und 2 Stunden bei 20-25° nachgerührt. Das Reaktionsgemisch wird eingedampft, in Aethylacetat gelöst und mit Wasser gewaschen und die Aethylacetat-Lösung eingedampft. Der Rückstand (20 g)

wird an 400 g Kieselgel chromatographiert und mit Toluol (250 ml Fraktionen) eluiert. Aus den Fraktionen 28-36 erhält man das 2-Benzyloxy-3-(2,3-epoxypropyloxy)-pyridin als Oel.

b) 4,8 g 2-Benzyloxy-3-(2,3-epoxypropyloxy)-pyridin und 3,0 g 1-(4-Piperidyl)-imidazolidin-2-on werden in 50 ml Isopropanol gelöst und 15-18 Stunden bei 20-30° gerührt. Die ausgefallenen Kristalle werden abgenutscht, mit Aether gewaschen und ergeben das 1-{1-[2-Hydroxy-3-(2-benzyloxy-3-pyridyloxy)-propyl]-4-piperidyl}-imidazolidin-2-on vom Smp. 145-147°.

Beispiel 10

Ein Gemisch von 7,1 g 2-Methyl-4-(2,3-epoxypropyl)-indol und 5,9 g 1-(4-Piperidyl)-2-imidazolidinon wird in 125 ml Isopropylalkohol gelöst und 6 Stunden unter Rückfluss gekocht. Danach wird das Gemisch im Eisbad abgekühlt und abfiltriert. Das Kristallisat löst man in Isopropanol und kristallisiert evt. unter Zusatz von Tierkohle um. Das 4-{3-[4-(2-oxo-3-imidazolidinyl)-1-piperidyl]-2-hydroxy-1-propyloxy}-2-methyl-1H-indol hat einen Smp. von 208-210°.

Auf analoge Weise wird unter Verwendung von 1-(4-Piperidyl)-3-methyl-2-imidazolidinon das 4- 3-[4-(1-Methyl-2-oxo-3-imidazolidinyl)-1-piperidyl]-2-hydroxy-1-propyloxy}-2-methyl-1H-indol vom Smp. 184-185° hergestellt.

Auf analoge Weise wird unter Verwendung von 1-(4-Piperidyl)-2-benzimidazolinon das 4-{3-[4-(2-Oxo-benzimidazolidinyl)-piperidyl]-2-hydroxy-1-propyloxy}-2-methyl-1H-indol vom Smp. 172-174° hergestellt.

Das Ausgangsmaterial wird wie folgt hergestellt:

208 g N-Benzylpiperidon werden mit 8,5 g N-Methyl-aethylendiamin in 2000 ml Methanol mit 15 g Platin auf Kohle hydriert, der Katalysator abfiltriert und das Filtrat zur Trockne eingedampft. Noch anhaftende Feuchtigkeit wird mit Toluol abgetrieben. 173 g des zurückbleibenden N'-(1-Benzyl-4-piperidyl)-N''-methyl-äthylendiamins werden in einem Rührkolben mit 96,5 g N,N-Diisopropyläthylamin in 3000 ml Acetonitril vorgelegt. Dazu werden während 30 Minuten bei 0° - 5° 117,5 g Chlorameisensäure-phenylester zugetropft. Es entsteht eine dicke Suspension zu der 96,5 g N,N-Diisopropyläthylamin zugegeben werden. Anschliessend wird das Ganze 20 Stunden am Rückfluss erhitzt. Dann wird zur Trockne eingedampft, mit Toluol alles Flüchtige abgetrieben und der Rückstand in 1500 ml Essigester aufgenommen. Die organische Phase wird 6 mal mit je 500 ml 2N Natronlauge extrahiert, getrocknet und eingedampft. 150 g des zurückbleibenden Oels werden mit 500 ml Alkohol und 150 ml konz. Natronlauge 14 Stunden am Rückfluss erhitzt. Dann wird der Alkohol abdestilliert und der Rückstand zwischen 1000 ml Essigester und Eiswasser verteilt. Die wässerige Phase wird abgetrennt und die organische Phase 4 mal mit je 250 ml 2N Natronlauge extrahiert, getrocknet und destilliert. Das 1-(1-Benzyl-4-piperidyl)-3-methyl-2-imidazolidinon siedet bei 185°/0.18 mm.

127 g des erhaltenen Produktes werden in 1500 ml Methanol mit 39 g Palladium auf Kohle debenzyliert. Der Katalysator wird abfiltriert, das Filtrat eingedampft und der Rückstand destilliert. Das 1-(4-Piperidyl)-3-methyl-2-imidazolidinon siedet bei 121-124°/0,01 mm/Hg und hat

einen Smp. von 76 - 79°.

Beispiel 11

Ein Gemisch von 3,1 g 2-Methyl-4-(2,3-epoxy-propyl)-indol und 3,4 g 3-n-Butyl-1-(4-piperidyl)-2-imidazolidinon wird in 75 ml Isopropylalkohol durch leichtes Erwärmen gelöst und 18 Stunden bei Raumtemperatur gerührt. Die entstandene Suspension engt man unter vermindertem Druck auf ca. 40 ml ein, und kühlt im Eisbad ab. Das weisse Kristallisat filtriert man ab und erhält nach Umkristallisation aus Isopropylalkohol-Diäthyläther das 4-{3-[4-(1-n-Butyl-2-oxo-3-imidazolidinyl)-1-piperidyl]-2-hydroxy-1-propyloxy}-2-methyl-1H-indol vom Smp. 116-120°C.

Das als Ausgangsstoff verwendete 3-n-Butyl-1-(4-piperidyl)-2-imidazolidinon kann auf folgende Weise hergestellt werden:

Zu einer Lösung von 10,1 g 1-(4-Piperidyl)-imidazolidin-2-on und 6,06 g Triäthylamin in 120 ml Methylenchlorid tropft man unter Rühren 20 ml einer 50%-igen Chlorameisensäurebenzylester-Lösung in Toluol. Man rührt noch 2 Stunden weiter bei Raumtemperatur und filtriert dann das ausgefallene Triäthylamin-Hydrochlorid ab. Das Methylenchlorid-Filtrat wäscht man zweimal mit je 50 ml Wasser, trocknet es mit Natriumsulfat und dampft es dann ein. Durch Behandeln mit Toluol wird der Rückstand von noch anhaftender Feuchtigkeit befreit. Nach Anreiben mit Diäthyläther erhält man als weisses Kristallisat das 1-(1-Carbobenzoxy-4-piperidyl)-imidazolidin-2-on vom Smp. 133-135°C.

Zu einem auf 80°C erwärmten Gemisch von 2,4 g Natriumhydrid in 100 ml absolutem Dimethylformamid gibt man portionenweise unter Rühren 25 g 1-(1-Carbobenzoxy-4-piperidyl)-imidazolidin-2-on. Man rührt das Gemisch noch 2 Stunden weiter bei 80°C und lässt dann langsam 18,2 g n-Butyljodid zutropfen (stark exotherme Reaktion). Nach weiteren 2 Stunden Rühren bei 80°C wird das Reaktionsgemisch unter vermindertem Druck eingedampft und der Rückstand durch Behandeln mit Toluol von noch anhaftendem Dimethylformamid befreit. Das Reaktionsprodukt wird in 250 ml Essigsäureäthylester gelöst, zweimal mit je 50 ml Wasser gewaschen, getrocknet mit Natriumsulfat und zur Trockne eingedampft. Den Rückstand chromatographiert man an 1,5 kg Kieselgel, wobei ein Gemisch von Chloroform-Methylalkohol (95:5) als Eluiergemisch verwendet wird. Man erhält auf diese Weise, als farbloses Oel, das 3-n-Butyl-1-(1-Carbobenzoxy-4-piperidyl)-imidazolidin-2-on.

Ein Gemisch von 43 g 3-n-Butyl-1-(1-Carbobenzoxy-4-piperidyl)-imidazolidin-2-on, 100 ml Essigsäure und 89 ml einer [illegible]8%-igen Lösung von Bromwasserstoffsäure in Eisessig, wird 3 Stunden bei 75°C Reaktionstemperatur gerührt. Dann dampft man das Gemisch unter vermindertem Druck ein, löst den Rückstand in 100 ml Wasser und extrahiert ihn 3 mal mit je 100 ml Diäthyläther. Die wässerige saure Phase stellt man mit 2N-Natronlauge alkalisch und extrahiert sie 3 mal mit je 100 ml Chloroform. Die vereinigten Chloroformextrakte trocknet man mit Natriumsulfat und dampft sie ein. Das verbleibende Oel wird unter Vakuum fraktioniert destilliert. So erhält man das 3-n-Butyl-1-(4-piperidyl)-imidazolidin-2-on vom Kp. 155°C bei 0,[illegible] Torr.

Beispiel 12

Ein Gemisch von 3,1 g 2-Methyl-4-(2,3-epoxy-propyl)-1H-indol und 3,2 g 3-(2-Hydroxy-äthyl)-1-(4-piperidyl)-2-imidazolidin-on wird durch leichtes Erwärmen in 75 ml Isopropylalkohol gelöst und 18 Stunden bei Raumtemperatur gerührt. Danach wird dieses Gemisch unter vermindertem Druck zur Trockne eingedampft. Nach Kristallisation des Rückstandes aus Methylenchlorid-Diäthyläther, evt. unter Zusatz von Aktivkohle, erhält man das 4-{3-[4-(1-Hydroxyäthyl-2-oxo-3-imidazolidinyl)-1-piperidyl]-2-hydroxy-1-propyloxy}-2-methyl-1H-indol vom Smp. 158-162°.

Das als Ausgangsstoff verwendete 3-(2-Hydroxy-äthyl)-1-(4-piperidyl)-imidazolidin-2-on kann auf folgende Weise hergestellt werden:

Zu einer Lösung von 10,1 g 1-(4-Piperidyl)-imidazolidin-2-on und 6,06 g Triäthylamin in 120 ml Methylenchlorid tropft man unter Rühren 20 ml einer 50%-igen Chlorameisensäurebenzylester-Lösung in Toluol. Man rührt noch 2 Stunden weiter bei Raumtemperatur und filtriert dann das ausgefallene Triäthylamin-Hydrochlorid ab. Das Methylenchlorid-Filtrat wäscht man zweimal mit je 50 ml Wasser, trocknet es mit Natriumsulfat und dampft es dann ein. Durch Behandeln mit Toluol wird der Rückstand von noch anhaftender Feuchtigkeit befreit. Nach Anreiben mit Diäthyläther erhält man als weisses Kristallisat das 1-(1-Carbobenzoxy-4-piperidyl)-imidazolidin-2-on vom Smp. 133-135°C.

Ein Gemisch von 1,35 g Natriumhydrid in 70 ml Dimethylformamid und 14,2 g 1-(1-Carbobenzoxy-4-piperidyl)-imidazolidin-2-on wird während 4 Stunden bei 80°C gerührt. Danach wird eine Lösung von 11,7 g 2-Bromäthyl-2-

BAD ORIGINAL

tetrahydro-pyranyläther in 30 ml absolutem Dimethylformamid innert 15 Minuten zugetropft. Man rührt das Gemisch noch weitere 4 Stunden bei 80°C und dampft es dann unter vermindertem Druck ein. Der Rückstand wird durch Behandeln mit Toluol von noch anhaftendem Dimethylformamid befreit. Das Reaktionsprodukt löst man in Essigsäureäthylester, wäscht die Lösung zweimal mit je 50 ml Wasser, trocknet die Essigsäureäthylester-Phase mit Natriumsulfat und dampft sie ein. Das zurückbleibende Oel chromatographiert man an 1,5 kg Kieselgel, wobei ein Gemisch von Chloroform-Methylalkohol (95:5) als Eluiergemisch verwendet wird. Man erhält auf diese Weise, als leicht gelbliches Oel, das 1-(1-Carbobenzoxy-4-piperidyl)-3-[2-(2-tetrahydropyranyloxy)-äthyl]-imidazolidin-2-on.

Eine Lösung von 59,7 g 1-(1-Carbobenzoxy-4-piperidyl)-3-[2-(2-tetrahydropyranyloxy)-äthyl]-imidazolidin-2-on in 1200 ml absolutem Methanol wird unter Zusatz von 1 Moläquivalent Salzsäure und 6 g Palladium-Kohle Katalysator bis zur Aufnahme von 2 Moläquivalent Wasserstoff bei Raumtemperatur und Normaldruck hydriert. Danach wird der Katalysator abfiltriert und das Filtrat unter vermindertem Druck eingedampft. Der Rückstand wird durch Behandeln mit Toluol von noch anhaftender Feuchtigkeit befreit. Das Reaktionsprodukt chromatographiert man an 2 kg basischem Aluminiumoxyd wobei ein Gemisch von Chloroform-Methanol (85:15) als Eluiergemisch verwendet wird. Man erhält auf diese Weise, als viskoses Oel, das 3-(2-Hydroxyäthyl)-1-(4-piperidyl)-imidazolidin-2-on, das ohne zusätzliche Reinigung weiterverwendet wird.

Beispiel 13

Ein Gemisch von 4,1 g 2-Methyl-4-(2,3-epoxy-propyl)-1H-indol und 5,2 g 3-Benzyl-1-(4-piperidyl)-2-imidazolidinon wird durch leichtes Erwärmen in 100 ml Isopropylalkohol gelöst und 24 Stunden bei Raumtemperatur gerührt. Danach wird das Gemisch im Eisbad abgekühlt und der Niederschlag abfiltriert. Das Kristallisat löst man in Isopropylalkohol und kristallisiert evt. unter Zusatz von Tierkohle um. Das 4-{3-[4-(1-Benzyl-2-oxo-3-imidazolidinyl)-1-piperidyl]-2-hydroxy-1-propyloxy}-2-methyl-1H-indol hat einen Smp. von 97-100°C.

Das als Ausgangsstoff verwendete 1-Benzyl-3-(4-piperidyl)-imidazolidin-2-on kann auf folgende Weise hergestellt werden.

Ein Gemisch von 42,8 g Benzylamin und 120 ml Methyläthylketon wird unter Rühren und bei Eisbadkühlung vorgelegt. Dazu gibt man portionenweise 41 g 2-Brom-äthylamin-hydrobromid und kocht während 16 Stunden am Rückfluss. Danach wird das Gemisch im Eisbad abgekühlt und der entstandene Niederschlag abfiltriert. Das Kristallisat löst man in 150 ml Wasser und wäscht es zweimal mit je 50 ml Diäthyläther. Die Wasserphase stellt man mit konz. Ammoniaklösung alkalisch und extrahiert sie viermal mit je 100 ml Chloroform. Die vereinigten Chloroformextrakte werden getrocknet und unter vermindertem Druck zur Trockne eingedampft. Durch Behandeln mit Toluol wird der Rückstand von noch anhaftender Feuchtigkeit befreit. Nach fraktionierter Destillation des Rückstandes am Hochvakuum erhält man das 1-Benzyl-äthylendiamin vom Kp. 88-90°C bei 0,02 Torr.

Eine Lösung von 29,25 g 1-Benzyl-äthylendiamin und 36,85 g 1-Benzyl-4-piperidon in 100 ml absolutem Methylalkohol wird unter Zusatz von 2 g eines 5%-igen Platin-Kohle Katalysators und 0,49 g konz. chemisch reiner Schwefelsäure bis zur Aufnahme von 1 Moläquivalent Wasserstoff bei Raumtemperatur und Normaldruck hydriert. Danach wird der Katalysator abfiltriert und das Filtrat zur Trockne eingedampft. Den Rückstand löst man in 2 N Salzsäure und extrahiert dreimal mit je 75 ml Diäthyläther. Die saure Wasserphase wird mit konz. Ammoniaklösung alkalisch gestellt und fünfmal mit je 75 ml Chloroform extrahiert. Die vereinigten Chloroformphasen trocknet man mit Natriumsulfat und dampft sie unter vermindertem Druck zur Trockne ein. Durch Behandeln mit Toluol wird der Rückstand von noch anhaftender Feuchtigkeit befreit, und man erhält auf diese Weise als öligen Rückstand das 1-Benzyl-4-(2-benzylamino-äthylamino)-piperidin, das ohne weitere Reinigung weiterverwendet wird.

13,7 g des rohen 1-Benzyl-4-(2-benzylamino-äthylamino)-piperidins werden in einem Rührkolben mit 5,5 g N,N-Diisopropylamin in 170 ml Acetonitril vorgelegt. Dazu werden während 30 Minuten bei 0-5°C 7,3 g Chlorameisensäure-phenylester zugetropft. Es entsteht eine dicke Suspension zu der 5,5 g N,N-Diisopropyl-äthylamin zugegeben werden. Anschliessend wird das Gemisch 18 Stunden unter dem Rückflusskühler erhitzt. Dann wird das Gemisch unter vermindertem Druck eingedampft und danach mit Toluol alles Flüchtige abgetrieben. Den Rückstand löst man in 150 ml Essigsäureäthylester und extrahiert die Lösung viermal mit je 50 ml 2N Natronlauge. Die organische Phase wird getrocknet und eingedampft. Das zurückbleibende Oel wird mit 60 ml Aethylalkohol und 25 ml konz. Natronlauge 15 Stunden am Rückfluss erhitzt. Dann wird der Alkohol abdestilliert und der Rückstand zwischen 100 ml Essigsäureäthylester und Eiswasser verteilt. Die wässerige Phase trennt man ab und die organische Phase extrahiert man dreimal mit je 30 ml 2N Natronlauge. Anschliessend wird die Essigsäureäthylester-Phase getrocknet und eingedampft. Den Rückstand chromatographiert man an 500 g Kieselgel, wobei ein Gemisch von Chloroform-Methylalkohol (9:1) als Eluiergemisch verwendet wird. Man erhält so, das 1-Benzyl-3-(1-benzyl-4-piperidyl)-imidazolidin-2-on.

Ein Gemisch von 12 g 1-Benzyl-3-(1-benzyl-4-piperidyl)-imidazolidin-2-on, 120 ml 70%-iger wässeriger Methylalkohol-Lösung und 3,39 g konz. chem. reiner Salzsäure wird unter Zusatz von 2,4 g eines 5%igen Palladium-Kohle-Katalysators bis zur Aufnahme von 1 Moläquivalent Wasserstoff bei Raumtemperatur und Normaldruck hydriert. Danach wird der Katalysator abfiltriert und das Filtrat unter vermindertem Druck eingedampft. Den Rückstand stellt man mit konz. wässeriger Ammoniaklösung alkalisch und extrahiert ihn fünfmal mit je 50 ml Chloroform. Die vereinigten Chloroformextrakte trocknet man und dampft sie unter vermindertem Druck ein. Das zurückbleibende Kristallisat wird in einem Gemisch von Diäthyläther-Petroläther verteilt, abfiltriert und getrocknet. Man erhält so, das 1-Benzyl-3-(4-piperidyl)-imidazolidin-2-on vom Smp. 110 - 113°C.

Beispiel 14

Ein Gemisch von 2,65 g 2-Phenyl-4-(2,3-epoxy-propyl)-indol und 2,0 g 1-(4-Piperidyl)-2-imidazolidinon wird in 100 ml Isopropanol 24 Stunden bei Zimmertemperatur gerührt. Dann wird das in geringer Menge ausgefallene Harz abgetrennt, das Filtrat eingedampft und der Rückstand in Methanol mit Tierkohle behandelt. Das Gemisch wird filtriert, das Filtrat eingedampft und der Rückstand aus Isopropyl-alkohol-Essigester 1:4 umkristallisiert. Das 4-{3-[4-(2-Oxo-3-imidazolidinyl)-1-piperidyl]-2-hydroxy-1-propyloxy}-2-phenyl-1H-indol hat einen Smp. von 166 - 168°C.

Das Ausgangsmaterial wird wie folgt hergestellt:

23,0 g 2-Phenyl-4-hydroxy-indol werden in eine Lösung von 5,2 g Natronlauge in 250 ml Wasser eingetragen und die Suspension mit 17,2 g Epibromhydrin 24 Stunden bei Raumtemperatur gerührt. Das ausgefallene graue Harz wird abfiltriert, in Chloroform gelöst, die Lösung getrocknet, eingedampft und der Rückstand im Kugelrohr bei 100°/12 mm Hg von noch anhaftendem Epibromhydrin befreit. Das zurückbleibende Oel wird in Chloroform-Methanol 98:2 an Kieselgel chromatographiert. Das 2-Phenyl-4-(2,3-epoxy-propyl)-indol wird aus Isopropylalkohol-Petroläther umkristallisiert und hat einen Smp. von 89 - 90°C.

Das 2-Phenyl-4-hydroxy-indol wurde aus dem 4-Oxo-2-phenyl-4.5.6.7-tetrahydroindol durch Aromatisierung mit Palladium-Kohle in Diphenyläther synthetisiert und zeigt einen Smp. 120 - 123°C.

Beispiel 15

4,5 g 1- 3-[(2-Methyl-4-indolyloxy)-2-hydroxy-1-propyl] -4-(2-oxo-3-imidazolidinyl)-pyridinium-bromid werden in einem Gemisch von 25 ml Wasser und 25 ml Aethanol mit 2,0 g Platindioxyd bei 40° hydriert. Hierauf wird der Katalysator abfiltriert und das Filtrat im Vakuum eingedampft. Zum Rückstand wird 2N Natronlauge gegeben und die alkalische Phase mit Essigsäureäthylester ausgezogen. Dieses Extrakt wird getrocknet und verdampft. Der Rückstand wird aus Isopropanol unter Zusatz von Tierkohle umkristallisiert. Das 4-{3-[4-(2-Oxo-3-imidazolidinyl)-1-piperidyl]-2-hydroxy-1-propyloxy}-2-methyl-1H-indol hat einen Smp. von 208 - 210°C.

Das quaternäre Ausgangsmaterial wird durch Erhitzen von 3-(2-Methyl-4-indolyloxy)-2-hydroxy-1-brompropan mit 1-(4-Pyridyl)-2-imidazolidinon in Dimethylformamid bei 100° hergestellt.

Beispiel 16

34,6 g 1-{3-[(2-Methyl-4-indolyloxy)-2-hydroxy-1-propyl]}-4-(2-amino-äthylamino)-piperidin werden in einem Rührkolben mit 13,0 g N,N-Diisopropyläthylamin in 350 ml Acetonitril vorgelegt. Dazu werden während 30 Minuten bei 0-5°C 15,6 g Chlorameisensäure-phenylester zugetropft. Es entsteht eine dicke Suspension zu der 13,0 g N,N-Diisopropyläthylamin zugegeben werden. Anschliessend wird das Gemisch 18 Stunden unter Rückfluss erhitzt, anschliessend im Vakuum eingedampft und dann mit Toluol alles Flüchtige abgetrieben. Den Rückstand löst man in 300 ml Essigsäureäthylester und diese Lösung extrahiert man viermal mit je 100 ml 2N Natron-

BAD ORIGINAL

lauge. Die organische Phase wird getrocknet und eingedampft. Das zurückbleibende Oel wird mit 120 ml Aethanol und 50 ml konz. Natronlauge 15 Stunden unter Rückfluss erhitzt. Hierauf wird der Alkohol abdestilliert und der Rückstand zwischen 200 ml Essigsäureäthylester und Eiswasser verteilt. Die wässerige Phase trennt man ab und extrahiert die organische Phase dreimal mit je 60 ml 2N Natronlauge. Anschliessend wird die Essigsäureäthylesterphase getrocknet und eingedampft. Der Rückstand wird aus Isopropanol unter Zusatz von Tierkohle umkristallisiert. Das 4-{3-[4-(2-Oxo-3-imidazolidinyl)-1-piperidyl]-2-hydroxy-1-propyloxy}-2-methyl-1H-indol hat einen Smp. von 208-210°.

Das Ausgangsmaterial wird durch reduktive Alkylierung von 1-[3-(2-Methyl-4-indolyloxy)-2-hydroxy-1-propyl]-4-piperidon mit Aethylendiamin unter Palladium-Kohle-Katalyse in Methanol hergestellt.

Beispiel 17

Tabletten, enthaltend 20 mg 4-{3-[4-(2-oxo-3-imidazolidinyl)-1-piperidyl]-2-hydroxy-1-propyloxy}-2-methyl-1H-indol-hydrochlorid können z.B. wie folgt hergestellt werden:

Zusammensetzung (für 5000 Tabletten)

| | |
|---|---|
| 4-{3-[4-(2-oxo-3-imidazolidinyl)-1-piperidyl]-2-hydroxy-1-propyloxy}-2-methyl-1H-indol-hydrochlorid | 100 g |
| Milchzucker | 150 g |
| Weizenstärke | 150 g |
| Kolloidale Kieselsäure | 25 g |
| Talk | 25 g |
| Magnesiumstearat | 5 g |
| Wasser | q.s. |

Das 4-{3-[4-(2-oxo-3-imidazolidinyl)-1-piperidyl]-2-hydroxy-1-propyloxy}-2-methyl-1H-indol-hydrochlorid wird mit dem Milchzucker, der kolloidalen Kieselsäure und einem Teil der Weizenstärke gemischt und die Mischung durch ein Sieb getrieben. Ein weiterer Teil der Weizenstärke wird mit der fünffachen Menge Wasser auf dem Wasserbad verkleistert und das Pulvergemisch mit diesem Kleister durchgeknetet, bis eine schwachplastische Masse entsteht. Die Masse wird durch ein Sieb getrieben, getrocknet und das trockene Granulat nochmals gesiebt. Darauf werden die restliche Weizenstärke, der Talk und das Magnesiumstearat zugemischt und das Gemisch zu Tabletten (mit Bruchrille) von 0,1 g Gewicht verpresst.

Patentansprüche

1. N-Aryl-diazacyclen der Formel

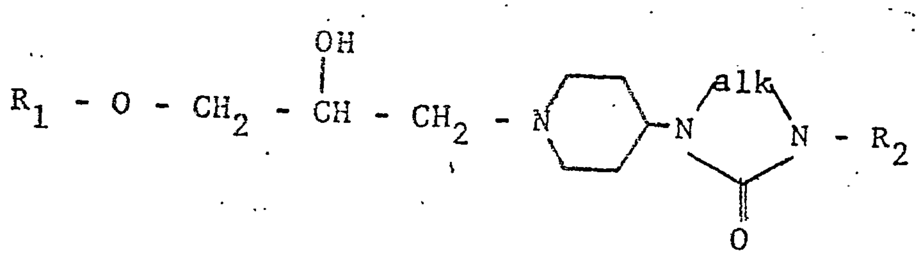

(I)

worin $R_1$ einen gegebenenfalls substituierten Heteroarylrest darstellt, $R_2$ Wasserstoff oder einen gegebenenfalls substituierten aliphatischen, cycloaliphatischen, cycloaliphatisch-aliphatischen oder araliphatischen Kohlenwasserstoffrest oder einen Acylrest bedeutet, und alk für Niederalkylen steht, das die beiden Stickstoffatome durch 2 oder 3 Kohlenstoffatome voneinander trennt, oder für einen gegebenenfalls substituierten 1,2-Phenylenrest steht, und deren Salze.

2. Verbindungen der Formel I gemäss Anspruch 1, worin $R_1$ gegebenenfalls substituiertes, monocyclisches Heteroaryl oder Benzoheteroaryl mit 5 bis 6 Ringgliedern und 1 oder 2 Ringstickstoffatomen im Heteroarylrest bedeutet, wobei Substituenten des Restes $R_1$ gegebenenfalls substituiertes Niederalkyl oder gegebenenfalls substituiertes Phenyl oder gegebenenfalls veräthertes oder verestertes Hydroxy oder Mercapto, oder Nitro sind,

$R_2$ für Wasserstoff oder gegebenenfalls substituiertes Niederalkyl, Cycloalkyl, Cycloalkylniederalkyl oder Phenylniederalkyl steht, wobei Substituenten dieser Reste gegebenenfalls substituiertes Niederalkyl oder gegebenenfalls veräthertes oder verestertes Hydroxy oder Mercapto sind, oder für Niederalkanoyl oder Niederalkoxycarbonyl steht, und alk die im Anspruch 1 gegebene Bedeutung hat, und deren Salze.

3. Verbindungen der Formel I gemäss Anspruch 1, worin $R_1$ gegebenenfalls durch Niederalkyl, Niederalkoxy, Niederalkylthio, Halogen mit Atomnummer bis zu 35 und/oder Nitro substituiertes monocyclisches Monoazaaryl oder Diazaaryl mit sechs Ringgliedern darstellt, $R_2$ für Wasserstoff, Niederalkyl, Cycloalkyl oder gegegebenenfalls im Phenylteil durch Niederalkyl, Niederalkoxy und/oder Halogen mit Atomnummer bis zu 35 substituiertes Phenylniederalkyl steht, und alk Niederalkylen mit 2-3 Kohlenstoffatomen bedeutet, das die beiden Stickstoffatome durch 2-3 Kohlenstoffatome trennt, und deren Salze.

4. Verbindungen der Formel I gemäss Anspruch 1, worin $R_1$ für gegebenenfalls durch Niederalkyl, Niederalkoxy, Niederalkylthio, Halogen mit Atomnummer bis zu 35, und/oder Nitro substituiertes 2-Pyrazinyl, Pyridyl oder Indolyl steht, $R_2$ Wasserstoff, Niederalkyl bedeutet, und alk Niederalkylen mit 2-3 Kohlenstoffatome bedeutet, das die beiden Stickstoffatome durch 2-3 Kohlenstoffatome trennt, und deren pharmazeutisch annehmbare Salze.

5. Verbindungen der Formel I gemäss Anspruch 1, worin $R_1$ für 4-Indolyl, 2-Pyrazinyl oder Pyridyl steht, das in ortho-Stellung zum Verknüpfungskohlenstoffatom und/oder Stickstoffatom gegebenenfalls durch Niederalkyl, Niederalkoxy, Niederalkylthio, Halogen mit Atomnummer bis zu 35, oder Nitro substituiert sein kann und gegebenenfalls weitere Substituenten dieser Art enthalten kann, $R_2$ für Wasserstoff, Niederalkyl oder Phenylniederalkyl steht, und alk Aethylen darstellt, und deren pharmazeutisch annehmbaren Salze.

6. Verbindungen der Formel I gemäss Anspruch 1, worin $R_1$ für 2-Pyrazinyl steht, das in 3-Stellung gegebenenfalls durch Niederalkyl, Niederalkoxy, Niederalkylthio, Halogen, mit Atomnummer bis zu 35, oder Nitro substituiert ist, $R_2$ Wasserstoff oder Niederalkyl bedeutet, und alk Aethylen darstellt, und deren pharmazeutisch annehmbaren Salze.

7. 1-{1-[2-Hydroxy-3-(3-methoxy-2-pyrazinyloxy)-propyl]-4-piperidyl}-imidazolidin-2-on und deren pharmazeutisch annehmbaren Salze.

8. 4-{3-[4-(2-oxo-3-imidazolidinyl)-1-piperidyl]-2-hydroxy-1-propyloxy}-2-methyl-1H-indol und deren pharmazeutisch annehmbaren Salze.

9. 4-{3-[4-(1-Methyl-2-oxo-3-imidazolidinyl)-1-piperidyl]-2-hydroxy-1-propyloxy}-2-methyl-1H-indol und deren pharmazeutisch annehmbaren Salze.

10. Die Verbindungen der Ansprüche 1-9 zur Verwendung als Pharmazeutika.

11. Pharmazeutische Präparate enthaltend eine der in den Ansprüchen 1-9 beanspruchten Verbindungen.

12. Verwendung der Verbindungen der Ansprüche 1-9 zur Herstellung von pharmazeutischen Präparaten.

13. Verfahren zur Herstellung von Piperidino-propanolen der Formel :

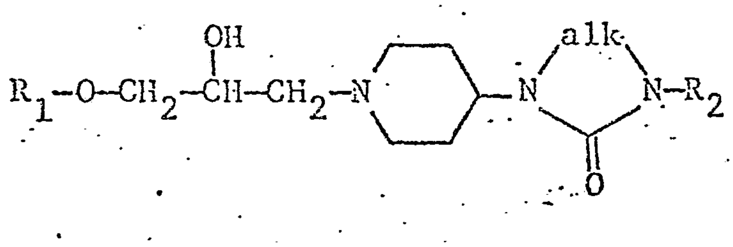

(I)

worin $R_1$ einen gegebenenfalls substituierten Heteroarylrest darstellt, $R_2$ Wasserstoff oder einen gegebenenfalls substituierten aliphatischen, cycloaliphatischen, cycloaliphatisch-aliphatischen oder araliphatischen Kohlenwasserstoffrest oder einen Acylrest bedeutet, und alk für Niederalkylen steht, das die beiden Stickstoffatome durch 2 oder 3 Kohlenstoffatome voneinander trennt, oder für einen gegebenenfalls substituierten 1,2-Phenylenrest steht, oder Salzen davon, dadurch gekennzeichnet, dass man

a) eine Verbindung der Formel

$$R_1 - O - X_1 \qquad (II)$$

oder ein Salz davon mit einer Verbindung der Formel

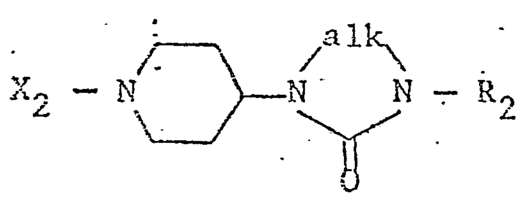

(III)

oder einem Salz davon umsetzt, worin einer der Reste $X_1$ und $X_2$ Wasserstoff bedeutet, und der andere dem Rest der Formel

$$-CH-\overset{X_3}{CH}-CH_2-X_4 \qquad (IV)$$

entspricht, worin $X_3$ für eine freie Hydroxylgruppe steht, und $X_4$ eine reaktionsfähige veresterte Hydroxylgruppe bedeutet, oder worin $X_3$ und $X_4$ zusammen eine Epoxygruppe bilden, oder

b) in einer Verbindung der Formel

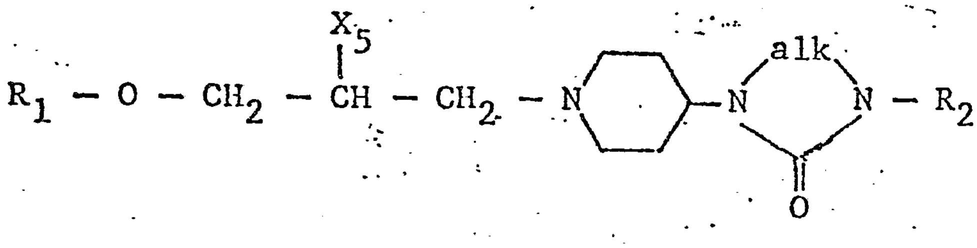

(VI) ,

worin $X_5$ einen in Hydroxy überführbaren Rest darstellt, $X_5$ in Hydroxy überführt, oder

c) in einer Verbindung der Formel

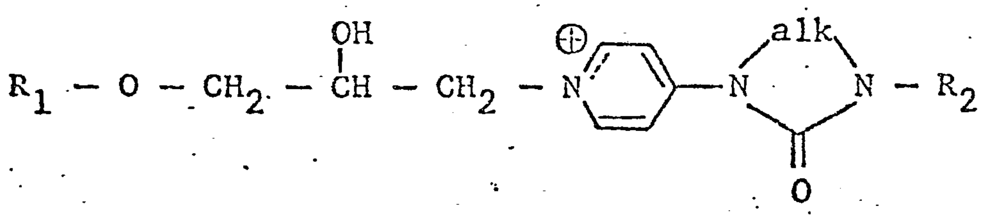

· $X^{\ominus}$

(VII)

worin $X^{\ominus}$ ein Anion bedeutet, den Pyridiniumring zum Piperidinring reduziert, oder

d) in einer Verbindung der Formel

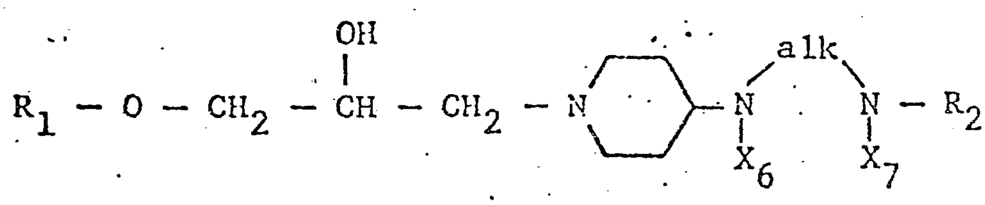

(XII)

worin einer der Reste $X_6$ und $X_7$ für Wasserstoff steht und der andere den Acylrest eines Kohlensäurehalbderivates

bedeutet, den 2-Oxo-1,3-diazacycloalkanring durch Ringschluss bildet, oder

c) in einer Verbindung der Formel

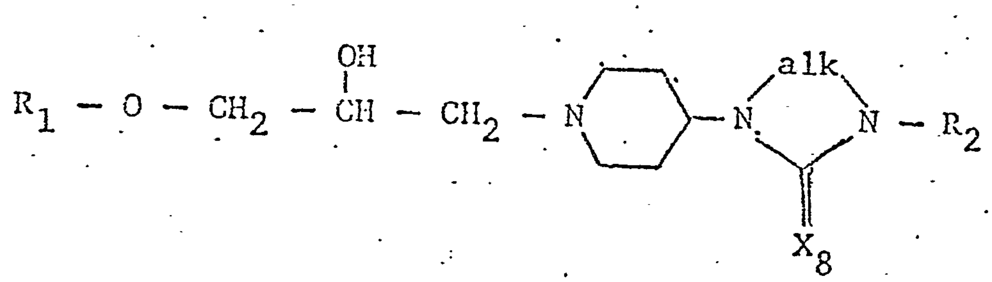

(XIII)

worin $X_8$ einen in Oxo überführbaren Iminorest darstellt, $X_8$ in Oxo überführt, und, wenn erwünscht eine erhältliche Verbindung in eine andere Verbindung der Formel I umwandelt, und/oder, wenn erwünscht, eine erhältliche freie Verbindung in ein Salz überführt, und/oder, wenn erwünscht, ein erhältliches Salz in die freie Verbindung oder in ein anderes Salz überführt, und/oder, wenn erwünscht, ein erhältliches Isomerengemisch in die Isomeren auftrennt.

0000485

Europäisches Patentamt

# EUROPÄISCHER RECHERCHENBERICHT

Nummer der Anmeldung
EP 78 10 0308

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| P | NL - A - 77 00530 (CIBA GEIGY)<br>* Patentansprüche; Seiten 36-40 *<br>-- | 1-13 | C 07 D 401/14<br>A 61 K 31/00<br>// 67 D 213/69<br>67 D 209/08<br>67 D 213/65 |
| A | NL - A - 73 11514 (CIBA GEIGY)<br>---- | | |

RECHERCHIERTE SACHGEBIETE (Int. Cl.²)

C 07 D 401/14
A 61 K 31/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| Den Haag | 08-10-1978 | CREMERS |